# EUROPEAN PATENT SPECIFICATION

(11) **EP 2 252 322 B1**
(45) Date of publication and mention of the grant of the patent: **12.10.2011**
(21) Application number: 09706841.5
(22) Date of filing: 30.01.2009
(51) Int. Cl.: A61K 38/20, A61P 35/00

(54) **VACCINE COMPOSITIONS**
IMPFSTOFF-ZUSAMMENSETZUNGEN
COMPOSITIONS VACCINALES

(30) Priority: 31.01.2008 WO PCT/EP2008/000780
(43) Date of publication of application: 24.11.2010
(73) Proprietor: AGIRx Limited, Chalvington East Sussex BN27 3TD (GB)
(72) Inventor: MACKIEWICZ, Andrzej, PL-61-051 Poznan (PL)
(74) Representative: Zwicker, Jörk
(86) International application number: PCT/EP2009/000626
(87) International publication number: WO 2009/095261

## Description

### FIELD OF THE INVENTION

The invention relates to tumour therapy. In particular, the present invention relates to vaccine compositions comprising allogenic cells modified with hypercytokines for the treatment of cancer in general and for the treatment of melanoma in particular.

### BACKGROUND OF THE INVENTION AND STATE OF THE ART

### Cytokines and cytokine receptors

IL-11 together with IL-6, Leukaemia Inhibitor Factor (LIF), Oncostatin M (OSM), Ciliary Neutrophic Factor (CNTF), Cardiotrophin 1 (CT-I) belongs to the family of hemopoietic cytokines (named IL-6-type or gp130 cytokines), which share structural similarity and a common receptor subunit (gp130) (Bazan et al., 1990). Although, each of the IL-6-type cytokines requires a specific (unique) receptor complex, at least one molecule of gp130 is always engaged. Initially a ligand (IL-6, IL-11, CNTF) binds specifically to its α non-signaling receptor subunit and next recruits the signaling receptor chain. IL-6 and IL-11 use a gp130 homodimer for transducing the signal, while LIF, CNTF, CT-I utilize a heterodimer gp130/LIFR. OSM either recruits gp130/OSMR or gp130/LIFR heterodimers (reviewed in Heinrich et al., 2003, Bravo et al., 2000).

The tertiary structure of IL-6-type cytokines has been intensely investigated during recent years. Crystal structures have been determined for LIF (Robinson et al., 1994), CNTF (McDonald et al., 1995), IL-6 (Somers et al., 1997) and OSM (Deller et al., 2000). These studies revealed that each ligand exhibits the long chain four-helix bundle topology, which comprises four tightly packed α-helices (named A, B, C and D) ranging from 15 to 22 amino acids in length. The helices are connected in an up-up-down-down arrangement by the polypeptide loops. The A-B and C-D loops are relatively long as they connect parallel helices, whereas the B-C loop is shorter as it connects a pair of antiparallel helices. Detailed structural analysis and mutagenesis studies of IL-6-type cytokines have identified three receptor binding sites (termed I, II, III), which seem to be conserved among the gp130 family (reviewed in Bravo et al., 2000). Site I, which enables ligand to bind to its non-signaling receptor, is formed by amino acids from the C- terminal part of the A-B loop and the C-terminal residues of the D helix. Site II seems to be a universal gp130 binding site for all members of IL-6-type cytokines and consists of exposed residues on helices A and C. Site III is composed of an N-terminal half of helix D, the N-terminal part of the A-B loop and amino acid residues of the end of the C-D loop. This site is always occupied by the second signaling receptor: gp130, LIFR or OSMR, depending upon the identity of the ligand.

The receptors involved in IL-6-type cytokine signaling belong to the type I membrane proteins. They possess an extracellular N-terminus and one transmembrane domain (with the exception of CNTFR, which is linked to the membrane by a lipid anchor (Davis et al., 1991). Because of a common structural motif in their extracellular region, they are classified as cytokine receptor class I family (Bazan et al., 1990). This family is characterized by the presence of at least one cytokine binding homology domain (CHD) consisting of two fibronectin-type-III-like domains (FNIII) termed D2 and D3. The CHD is composed of approximately 200 amino acids, with four positionally conserved cysteine residues at the N-terminal domain and a characteristic conserved Trp-Ser-X-Trp-Ser (WSXWS) motif at the C-terminal domain. Additionally each receptor subunit contains an Ig-like domain, which is located at the N-terminus of the membrane- proximal CHD. The IL-6-type receptors are divided into two groups: α and β subunits. Receptors α (for IL-6, IL-11 and CNTF) are not involved in signal transduction. Subunits β, the signal transducing receptor chains, contain a considerably larger cytoplasmic part than α subunits and have three membrane-proximal FNIII domains that may play some role such as in stabilization and/or in orientation of the transmembrane receptor dimers (reviewed in Bravo et al., 2000, Heinrich et al., 2003). Besides the membrane bound IL-6-type receptor subunits, their soluble forms were found in biological fluids (reviewed in Marz et al., 1999). They are formed either by limited proteolysis (shedding) of membrane-bound receptors or by translation from differently spliced mRNA.

### Fusion proteins of cytokines and cytokine receptors

In order to increase and modify potential bioactivity of some molecular agents, the idea of linkage of two soluble naturally existing components has been postulated. Such fusion proteins have already been described. The separately encoded subunits of IL-12 (p35 and p40) have been connected by a polypeptide linker (Lieschke et al., 1997). Hyper-IL-6 is another example of a new designer agent, which consists of D2 and D3 domain of IL-6 R α chain connected to IL-6 via polypeptide linker (Fischer et al., 1997 and WO 97/32891). In the case of IL-6, it was observed that the effective concentration of IL-6 and sIL-6 R, which is needed for the stimulation of cells which lack membrane IL-6 R is very high (Rose-John et al., 1990). Furthermore, the average half-life of the IL-6/sIL-6 R complex might be shorter than the time needed to assemble the IL-6/sIL-6 R/gp130 complex (Wells et al., 1996). The stability of IL-6/sIL-6 R complex was enhanced by linking both components in order to create a fusion protein (Hyper-IL-6) (WO 97/32891). Hyper-IL-6 can directly bind to its signal transducing receptor subunit and enhance IL-6 activity. Hyper-IL-6 is a fully active fusion protein, which mediates response at 100 to 1000-fold lower dose compared to the combination of soluble IL-6 and sIL-6 R molecules (Fischer et al., 1997). In analogy, another superagonist has been designed for IL-6-type family named IL-11/R-FP (Pflanz et al., 1999). IL-11/R-FP was created by covalently linking D2 and D3 domains of IL-11 R (position L/109 - G/318) with IL-11 (position P/29 - L/199) using a 21 amino acid linker and demonstrated 50-fold higher activity *in vitro* than the combination of IL-11 and sIL-11 R. However, this construct was composed of truncated segments of the human IL-11 R and IL-11 and, thus, lacks naturally existing parts of the respective receptor and cytokine. Moreover, the artificial linker used is no naturally occurring sequence, which contributes to the immunogenicity of IL-11/R-FP when used for treatment of human patients. Another sIL-11 R IL-11 fusion protein comprising larger parts of the full length sIL-11 was described in WO 2005/113591 and which exhibited advantageous properties if compared to IL-11/R-FP.

WO 99/02552 A2 (Yeda Research and Development Co. Ltd. (Revel M. et al.) "Chimeric interleukin-6 soluble receptor/ligand protein, analogs thereof and uses thereof", published 21 Jan 1999) relates to chimeric proteins comprising a fusion protein product of sIL-6R and IL-6 and biologically active analogs of such proteins. In these chimeric proteins sIL-6R may be directly fused to IL-6 or via specific linker peptides. WO 99/02552 A2 further discusses a potential use of said chimeric proteins or analogs as inhibitors of cancer cells. It is also contemplated to use said chimeric proteins for the preparation of a medicament for treating mammalian cancers, for enhancement of bone marrow transplantation, for increasing hematopoeisis, or for treating liver or neurological disorders. The specific fusion proteins sIL-6R/IL-6 and sIL-6RδVal/IL-6 produced and examined in the Example section of WO 99/02552 have also been studied in an article by Chebath et al. (1997).

A review article by Kallen K.J. et al. (1997) discusses the potential therapeutic applications of interleukin-6 hyperagonists and antagonists. Said therapeutic applications comprise haematologic disorders, solid malignancies, cardiac ischaemia and transplantation, bone disease, glomerulonephritis and amyloidosis, acquired immunodeficiency syndrome, rheumatic disorders, autoimmunity, bums and major trauma, anaemia, expansion of immature haematopoietic stem cells in bone marrow transplantation and tumour therapy, inducing thrombopoiesis and liver regeneration.

Fusion proteins as those described above which comprise a cytokine and its physiological receptor are sometimes also called "hypercytokines" due to their high activity at lower doses as compared to the individual cytokine and/or a mixture of the cytokine with its soluble receptor.

### Tumour vaccines

The concept of therapeutic cancer vaccines is based on the knowledge that adaptive immunity can be primed and activated to specifically recognize and kill tumour cells. For the last 25 years, several vaccine studies have demonstrated immunological and clinical responses in selected patients, e.g. in renal cell cancer (Kubler & Vieweg 2006). Following the discovery of tumour-associated antigens or dendritic cells (DCs) along with the progress made in molecular biology and biotechnology, which provided recombinant cytokines and gene delivery systems, several strategies of tumour vaccination were proposed: tumour cell-based vaccines consisting of tumour cells admixed with a particular adjuvant (e.g., bacillus Calmette-Guerin, *Corynebacterium parvum* or IFNs); genetically modified tumour vaccines based on tumour cells expressing genes encoding immunostimulatory factors; and DCs modified with tumour-derived RNA, loaded with peptides/tumour lysates or fused with tumour cells.

The inventors of the present application studied tumour vaccines designed for mass scale production. Such tumour vaccines consist of established allogenic tumour cells which are irradiated and injected into tumour-bearing patients. In studies with tumour cells genetically modified to express hypercytokines the inventors surprisingly found that a composition comprising two different genetically modified tumour cell lines has a synergistic effect at least on the IL-2 and INF-γ production of peripheral blood lymphocytes. This increased production of IL-2 and INF-γ causes a beneficial shift of the immune response towards a Th1 immune response which is connected with cytotoxic activity. The compositions of the present invention comprising a first and a second allogenic cell line genetically modified to express the same or different hypercytokines will therefore be better suited as medicaments for the treatment of tumours as those known from the prior art.

### SUMMARY OF THE INVENTION

According to a first aspect the present invention relates to a composition comprising (1) one or more first tumour cells modified to express a first hyper-cytokine, and (2) one or more second tumour cells modified to express a second hyper-cytokine, wherein the one or more second cells are different from the one or more first cells and of a different HLA type.

According to a second aspect the present invention relates to a composition according to the first aspect for use in medicine.

According to a third aspect the present invention relates to a pharmaceutical composition comprising a composition according to the first or the second aspect additionally comprising pharmaceutically acceptable diluents, carriers, excipients, fillers, binders, lubricants, glidants, disintegrants, adsorbents, and/or preservatives.

According to a fourth aspect the present invention relates to the composition according to the first or second aspect or the pharmaceutical composition according to the third aspect for the treatment or prevention of cancer.

In a fifth aspect the present invention relates to the use of a composition according to the first or second aspect for the preparation of a pharmaceutical composition for the treatment or prevention of cancer.

### BRIEF DESCRIPTION OF THE DRAWINGS

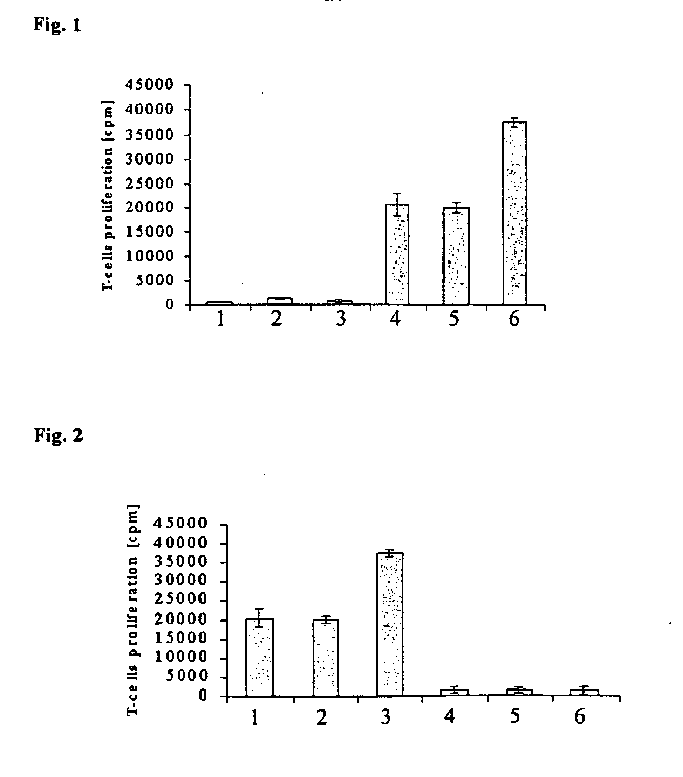

**Fig. 1** shows the T-cell proliferative response in an allogenic mixed tumour-lymphocyte reaction (AMTLR).
   Column 1: spontaneous T-cell proliferation, i.e. without tumour cells;
   column 2: spontaneous T-cell proliferation (without tumour cells) in the presence of IL-6;
   column 3: spontaneous T-cell proliferation (without tumour cells) in the presence of H6;
   column 4: T-cell proliferation in response to allogenic tumour cells;
   column 5: T-cell proliferation in response to allogenic tumour cells in the presence of IL-6;
   column 6: T-cell proliferation in response to allogenic tumour cells in the presence of H6.
**Fig. 2** shows the effect of anti-IL-2 antibody on the T-cell proliferation in an allogenic mixed tumour-lymphocyte reaction (AMTLR).
   Column 1: T-cell proliferation in response to allogenic tumour cells;
   column 2: T-cell proliferation in response to allogenic tumour cells in the presence of IL-6;
   column 3: T-cell proliferation in response to allogenic tumour cells in the presence of H6;
   column 4: T-cell proliferation in response to allogenic tumour cells with IL-2 neutralization;
   column 5: T-cell proliferation in response to allogenic tumour cells in the presence of IL-6 with IL-2 neutralization;
   column 6: T-cell proliferation in response to allogenic tumour cells in the presence of H6 with IL-2 neutralization.
**Fig. 3** shows the cytokine secretion by Mich1-H6 and Mich2-H6 cells expressed as MFI and pg/ml.
**Fig. 4** shows the cytokine secretion by PBLC isolated from healthy individuals expressed as MFI and pg/ml.
**Fig. 5** shows results from the stimulation of PBLC cytokine production by Mich1-H6 cells, by Mich2-H6 cells, and by a combination of Mich1-H6 and Mich2-H6 cells. The results are expressed as MFI and in pg/ml.

### DETAILED DESCRIPTION

### Definitions

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Kölbl, H. eds. (1995), Helvetica Chimica Acta, CH-4010 Basel, Switzerland).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety. Nothing herein is to be construed as an admission that the invention is not entitled to antedate such disclosure by virtue of prior invention.

The term "hyper-cytokine" refers to a fusion protein comprising, essentially consisting or consisting of (a) a soluble part of a cytokine receptor, and (b) a cytokine which can bind under physiological conditions to said soluble part of a cytokine receptor and an optional peptide linker positioned between the soluble cytokine receptor and the cytokine. In preferred embodiments, said cytokine is GM-CSF, IL-6, IL-11, IL-15, anti-TGF, EPO, interferon, LIF, OSM, CNTF, CT-1. If the cytokine is located N-terminally with respect to the cytokine receptor it is preferred that the cytokine still comprises its secretion signal, which will be cleaved during maturation of the protein, i.e. the mature hypercytokine protein will not comprise the secretion signal. If the cytokine is located C-terminally with respect to the cytokine receptor it is preferred that the cytokine does not comprise its secretion signal. The term "soluble cytokine receptor" refers to a soluble fragment of the cytokine receptor, e.g. which lacks most or all of the membrane-spanning part and the cytosolic part and comprises most or all of the extracellular part of the cytokine receptor, as for example sIL-6R and sIL-11R. The receptor fragment is soluble, if it is not or essentially not inserted into the membrane of a mammalian cell, preferably a human cell, expressing the receptor fragment. If the cytokine receptor is located N-terminally with respect to the cytokine it is preferred that the cytokine receptor still comprises its secretion signal, which will be cleaved during maturation of the protein, i.e. the mature hypercytokine protein will not comprise the secretion signal. If the cytokine receptor is located C-terminally with respect to the cytokine it is preferred that the cytokine receptor does not comprise its secretion signal. As indicated above the hyper-cytokine optionally may comprise a peptide linker positioned between the cytokine receptor and the cytokine. Preferably, said peptide linker has a low immunogenicity or is non-immunogenic. More preferably, said peptide linker is non-immunogenic to human beings. In preferred embodiments, the soluble cytokine receptor is located at the amino-terminal part of the hyper-cytokine and the cytokine is located at the carboxy-terminal part of the hyper-cytokine.

The term "hyper-cytokine activity" refers to the activity of the fusion protein. While particularly preferred hypercytokines have based on the same molar amount a 100- to 1000-fold higher activity in the same assays as the cytokine on which they are based or as a mixture of the cytokine and the cytokine receptor, i.e. the unfused parts forming the hypercytokine, not every hypercytokine will show such a dramatic improvement, which will depend among others on the length of the parts of cytokine and soluble cytokine receptor included and the length of the protein linker, if any, present. Numerous assays are known to assess the activity of the respective cytokine which forms the basis for a hypercytokine that can be employed in the present invention. If the respective hypercytokine has at least 10-fold the activity of the natural occurring cytokine on which it is based (at the same molar amount) or as a mixture of the unfused parts of the cytokine and the soluble part of the cytokine receptor it is considered within the meaning of this invention to exhibit hypercytokine activity. Preferably, it has at the same molar amount at least 20-fold, 30-fold, 40-fold, 50-fold, 60-fold, 70-fold, 80-fold, 90-fold, 100-fold, 150-fold, 200-fold, 250-fold, 300-fold, 350-fold, 400-fold, 450-fold, 5000-folde, 550-fold, 600-fold, 650-fold, 700-fold, 750-fold, 800-fold, 850-fold, 900-fold, 950-fold or 1000-fold the activity of the cytokine on which it is based or of a combination of the cytokine and the soluble cytokine receptor. Suitable assay systems include, e.g. for IL-6 hypercytokine the induction of proliferation of BAF-3/cells as described in Fischer M. *et al.* (1997).

The expression "at least 90% sequence identity" used throughout the specification preferably refers to a sequence identity of at least 90%, at least 91%, at least 92%, at least 93%, at least 94%, at least 95%, at least 96%, at least 97%, at least 98%, or at least 99% to the respective reference polypeptide. In case where the reference sequence is not specified in comparison to which the sequence identity percentage is to be calculated, the sequence identity is to be calculated with reference to the longer of the two sequences to be compared. If the reference sequence is indicated the sequence identity is determined on the basis of the full length of the sequence indicated by SEQ ID. For example, a peptide sequence consisting of 21 amino acids compared to the amino acids of full length IL-6 according to SEQ ID NO: 2 may exhibit a maximum sequence identity percentage of 9.9% (21 : 212) while a sequence with a length of 106 amino acids may exhibit a maximum sequence identity percentage of 50% (106:212).

The similarity of nucleotide and amino acid sequences, i.e. the percentage of sequence identity, can be determined via sequence alignments. Such alignments can be carried out with several art-known algorithms, preferably with hmmalign (HMMER package, http://hmmer.wustl.edu/) or with the CLUSTAL algorithm (Thompson J.D. et al., 1994) available e.g. on http://www.ebi.ac.uk/clustalw/ or on http://npsa-pbil.ibcp.fr/cgi-bin/npsa_automat.pl?page=/NPSA/npsa_clustalw.html. Preferred parameters used are the default parameters as they are set on http://www.ebi.ac.uk/clustalw/index.html#. The grade of sequence identity (sequence matching) may be calculated using e.g. BLAST, BLAT or BlastZ (or BlastX). Preferably, sequence matching analysis may be supplemented by established homology mapping techniques like Shuffle-LAGAN (Brudno M., 2003) or Markov random fields. When percentages of sequence identity are calculated in the context of the present invention, these percentages are to be calculated in relation to the full length of the longer sequence, if not specifically indicated otherwise.

A "peptide linker" in the context of the present invention refers to an amino acid sequence of between 1 and 100 amino acids. In preferred embodiments, a peptide linker according to the present invention has a minimum length of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids. In further preferred embodiments, a peptide linker according to the present invention has a maximum length of 100, 95, 90, 85, 80, 75, 70, 65, 60, 55, 50, 45, 40, 35, 34, 33, 32, 31, 30, 29, 28, 27, 26, 25, 24, 23, 22, 21, 20, 19, 18, 17, 16, or 15 amino acids or less. In especially preferred embodiments, the above-indicated preferred minimum and maximum lengths of the peptide linker according to the present invention may be combined, if such a combination makes mathematically sense. In further preferred embodiments, the peptide linker of the present invention is non-immunogenic; in particularly preferred embodiments, the peptide linker is non-immunogenic to humans.

### Embodiments of the Invention

The present invention will now be further described. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

In a first aspect, the present invention provides a composition comprising, essentially consisting or consisting of:
(1) one or more first tumour cells modified to express a first hyper-cytokine, and
(2) one or more second tumour cells modified to express a second hyper-cytokine,
   wherein the one or more second cells are different from the one or more first cells and have a different HLA type than the first cells.

The second cells are different from the first cells, if the first and the second cells are derived from different cell lines and/or if the second cells carry a different genetic modification than the first cells, e.g. first and second cells have been modified to express different hypercytokines. In preferred embodiments the first and second cells, respectively, are derived from tissue of two different individuals, preferably from two different humans. It is preferred that the two tissues, preferably tumour tissues are of the same type. The term "tissue" as used herein refers to both solid tissue like, e.g. skin, liver, brain, kidney, lung, stomach, colon, bladder, or testes, as well as mobile cell populations like, e.g. lymphocytes, or stem cells. While it is possible that the cells are autologous or allogenic, it is particularly preferred that the first and/or the second cells are allogenic. The term "allogenic" characterizes the relation between the cells and a patient receiving the cells. Cells from a particular individual will be allogenic to any other patient, while they will be autologous to that particular individual. Allogenicity is a prerequisite for industrial large scale production of any cell based vaccine, since otherwise each cellular vaccine would have to be produced individually from cells isolated and cultured from the respective patient to be treated. Allogenic cells provide additional advantages, which include that allogenic cells tend to induce a stronger immune response in a patient than autologous cells.

The terms "one or more first cells" and "one or more second cells" as used in the present invention refer to an individual cell, to a clonal population of that cell and to an assortment of similar cells. Thus, in a preferred embodiment, wherein the cells are derived from primary tissue, preferably a primary tumour, the cells will not all be clonal, but will be composed of one, two, three or more clonal cell populations belonging to a particular cell and/or tumour type. In particular, tumour cells show a high genetic variability upon propagation and, thus, it is common that cells within one established cell line are not entirely identical genetically. These cells are an example of an assortment of similar cells. Another example are primary tumour cells originating from one tumour, which have undergone 1, 2, 3, 4, 5, 6, 7, 8, 9, 10 or more cycles of subcultivation *in vitro,* which will lead to selection of proliferating cell subtypes and, thus, to reduction of heterogeneity of the cell population, i.e. render the assortment of cells more similar. In an embodiment, wherein the first and/or second cells are derived from primary tissue, preferably the same type of primary tissue, in particular tumour tissue, the first and second cells are considered different, if they are derived from two different individuals, preferably from two different humans.

The term "modified to express" indicates that a gene encoding the respective hyper-cytokine has been stably introduced into the cell in a form which allows stable expression of the gene encoding the hypercytokine and, subsequently, production of the respective hypercytokine.

Preferably the gene encoding the hypercytokine is introduced into an expression vector for use in mammalian cells, which ordinarily include an origin of replication (as necessary, see below), a promoter located in front of the gene to be expressed, optionally an enhancer in trans, along with any necessary ribosome binding sites, RNA splice sites, polyadenylation site, and transcriptional terminator sequences. Such an expression vector may then be used to modify the cell to express the respective hypercytokine.

In a preferred embodiment the expression vector of the present invention comprises, essentially consists or consists of plasmids; phagemids; phages; cosmids; artificial chromosomes, in particular artificial mammalian chromosomes or artificial yeast chromosomes; knock-out or knock-in constructs; viruses, in particular adenovirus, vaccinia virus, attenuated vaccinia virus, canary pox virus, lentivirus (Chang and Gay, 2001), herpes virus, in particular Herpes simplex virus (HSV-1, Carlezon, *et al.,* 2000), baculovirus, retrovirus, adeno-associated-virus (AAV, Carter and Samulski, 2000), rhinovirus, human immune deficiency virus (HIV), filovirus, and engineered versions of above mentioned viruses (see, for example, Kobinger *et al.,* 2001); virosomes; "naked" DNA, liposomes; virus-like particles; and nucleic acid coated particles, in particular gold spheres. Particularly preferred are viral vectors like adenoviral vectors, lentiviral vectors, baculovirus vectors or retroviral vectors (Lindemann *et al.,* 1997, and Springer *et al.,* 1998). Examples of plasmids, which allow the generation of such recombinant viral vectors include pFastBacl (Invitrogen Corp., Carlsbad CA), pDCCMV (Wiznerowicz *et al.,* 1997) and pShuttle-CMV (Q-biogene, Carlsbad, California). In cases where an adenovirus is used as an expression vector, the coding sequences may be ligated to an adenovirus transcription/translation control complex, e.g., the late promoter and tripartite leader sequence. The hypercytokine gene may be inserted in the genome of an adenovirus by *in vitro* or *in vivo* recombination. Insertion in a non-essential region of the viral genome (e. g., region E1, E3, or E4) will result in a recombinant virus that is viable and capable of expressing the respective hypercytokine in infected cells. It is preferred that the viral vector used is modified to be replication incompetent in order to prevent that first and/or second cells modified to express the hypercytokine produce viral particles.

To allow stable expression of a transgene the expression vector either has to be provided with an origin of replication, which allows replication independent from the genome of the cell or has to be integrated into the genome of the first and/or second cells. In the first case the expression vector is maintained episomally. Suitable origins of replication may be derived from SV40 or other viral (e. g., Polyoma, Adeno, CMV, VSV, BPV) source. In the latter case, if the expression vector is integrated into the genome, e.g. a chromosome, it is not required to provide an origin of replication.

To direct expression of the hypercytokine the gene encoding it is operationally linked to a promoter and/or enhancer that is recognized by the transcriptional machinery of the cell. Suitable promoters may be derived from the genome of mammalian cells (e. g., metallothionein promoter) or from mammalian viruses (e.g., the adenovirus late promoter; the vaccinia virus 7.5K promoter or the cytomegalovirus promoter). The early and late promoters of SV40 virus are particularly useful because both are obtained easily from the virus as a fragment which also contains the SV40 viral origin of replication. Smaller or larger SV40 fragments may also be used, provided there is included the approximately 250 bp sequence extending from the *Hind*III site toward the *Bgl*II site located in the viral origin of replication. Further, it is also possible, and may be desirable, to utilize promoter or control sequences normally associated with the cytokine or cytokine receptor encoding polynucleotide on which the hypercytokine is based.

As used herein, "operatively linked" means incorporated into a genetic construct so that expression control sequences effectively control expression of a coding sequence of interest.

Specific initiation signals may also be required for efficient translation of hypercytokine coding sequences. These signals include the ATG initiation codon and adjacent sequences. Exogenous translational control signals, including the ATG initiation codon, may additionally need to be provided. One of ordinary skill in the art would readily be capable of determining this and providing the necessary signals. It is well known that the initiation codon must be in-frame (or in-phase) with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements and transcription terminators. In eukaryotic expression, one will also typically desire to incorporate into the transcriptional unit an appropriate polyadenylation site (e.g., 5'-AATAAA-3') if one was not contained within the original cloned segment. Typically, the poly A addition site is placed about 30 to 2000 nucleotides "downstream" of the termination site of the protein at a position prior to transcription termination.

As indicated above rather than using expression vectors that contain viral origins of replication, cells can be transformed with vectors controlled by appropriate expression control elements (e.g., promoter, enhancer, sequences, transcription terminators, polyadenylation sites, etc.), and a selectable marker. Following the introduction of foreign DNA, engineered cells may be allowed to grow for 1-2 days in an enriched medium, and are then switched to a selective medium. The selectable marker in the recombinant plasmid confers resistance to the selection and allows cells to stably integrate the plasmid into their chromosomes and grow to form foci which in turn can be cloned and expanded into cell lines.

A number of selection systems may be used including, but not limited to, the herpes simplex virus thymidine kinase (tk), hypoxanthine-guanine phosphoribosyltransferase (hgprt) and adenine phosphoribosyltransferase (aprt) genes, in tk-, hgprt- or aprt- cells, respectively. Also antimetabolite resistance can be used as the basis of selection for dihydrofolate reductase (dhfr), that confers resistance to methotrexate; gpt, that confers resistance to mycophenolic acid; neomycin (neo), that confers resistance to the aminoglycoside G-418; and hygromycin (hygro), that confers resistance to hygromycin.

In a preferred embodiment the expression vector used to transform, transfect or infect the cell to be modified comprises the gene encoding the selectable marker as one transcript with the gene encoding the hypercytokine. To ascertain the individual expression of the selectable marker and the hypercytokine an internal ribosome entry site (IRES) is placed between the two coding sequences.

The tumour cells to be included in the composition of the present invention are preferably propagated separately. Preferably they are propagated *in vitro* in one of two modes: as non-anchorage dependent cells growing in suspension throughout the bulk of the culture or as anchorage-dependent cells requiring attachment to a solid substrate for their propagation (i.e., a monolayer type of cell growth). The appropriate growth conditions are determined by the cell type and can be determined by the skilled person using routine experimentation.

In a preferred embodiment of the composition of the first aspect, the first and/or second hyper-cytokine is a fusion protein comprising, consisting essentially of or consisting of a soluble cytokine receptor and a cytokine. In preferred embodiments, the soluble cytokine receptor is independently selected from (a) the group consisting of sIL-6R, sIL-11R, sOSM-R, sCNTF-R, and sCT-I-R; or (b) a polypeptide exhibiting at least 90% sequence identity to a polypeptide according to (a); and the cytokine is independently selected from (c) the group consisting of IL-6, IL-11, OSM, CNTF, and CT-I; or (d) a polypeptide exhibiting at least 90% sequence identity to a polypeptide according to (c), and optionally a peptide linker between the soluble cytokine receptor and the cytokine, wherein the resulting fusion protein has hyper-cytokine activity. Preferably the arrangement is from the N-terminal end to the C-terminal end of the fusion protein as follows: soluble cytokine receptor- optional peptide linker- cytokine. To ascertain secretion of the expressed hypercytokine the hypercytokine comprises at least one natural or artificial secretion signal. Since all cytokines are secreted they naturally comprise such a secretion signal. Similar signalling peptides are also found in cytokine receptors. Preferably this secretion signal is located at the N-terminal end of the fusion protein. It will be cleaved during processing and/or secretion of the hypercytokine.

When referring to sIL-6R, sIL-11R, sOSM-R, sCNTF-R, and sCT-I-R the respective soluble parts of IL-6R, IL-11R, OSM-R, CNTF-R, and CT-I-R, preferably of human origin are meant, the sequence of which are indicated herein as SEQ ID NO: 1 for IL-6R and SEQ ID NO: 3 for IL-11R. The sequences of all other cytokine receptors can be accessed on NIH Genebank or EMBL databanks, e.g. for OSM-R (Genebank Acces.: NP_003990) and CNTF-R (Genebank Acces.: NP_001833). When referring to IL-6, IL-11, OSM, CNTF, and CT-I, preferably those of human origin are meant, the sequence of which are indicated herein as SEQ ID NO: 2 for IL-6 and SEQ ID NO: 4 for IL-11. The sequences of all other cytokines can be accessed on NIH or EMBL databanks, e.g. for OSM (Genebank Acces. NO: P13725) and CNTR (Genebank Acces.: NP_000605).

In one embodiment of the composition of the first aspect, the hyper-cytokine is a fusion protein comprising, consisting essentially of or consisting of:
(a) an IL-6R part exhibiting at least 90% sequence identity to human soluble IL-6 receptor (sIL-6R), wherein said sequence identity is calculated over the entire length of the polypeptide sequence from P113 to A323 of SEQ ID NO: 1,
(b) an IL-6 part exhibiting at least 90% sequence identity to human interleukin-6 (IL-6), wherein said sequence identity is calculated over the entire length of the polypeptide sequence from P29 to M212 of SEQ ID NO: 2, and
(c) an optional peptide linker;
   wherein the fusion protein has hyper-cytokine activity, preferably at least 10%, at least 20%, at least 30%, at least 40%, at least 50%, at least 60%, at least 70%, at least 80%, at least 90%, at least 100% or more of the activity of the Hyper-IL-6 fusion protein according to SEQ ID NO: 9, when tested in a relevant assay of IL-6 activity, e.g. the induction of proliferation of BAF-3/cells as described in Fischer M. *et al.* (1997).

In a further embodiment of the composition of the first aspect, the hyper-cytokine is a fusion protein comprising, consisting essentially of or consisting of:
(a) an IL-11R part exhibiting at least 90% sequence identity to human soluble IL-11 receptor (sIL-11R), wherein said sequence identity is calculated over the entire length of the polypeptide sequence from M1 to G365 of SEQ ID NO: 3,
(b) an IL-11 part exhibiting at least 90% sequence identity to human interleukin-11 (IL-11), wherein said sequence identity is calculated over the entire length of the polypeptide sequence from A19 to L199 of SEQ ID NO: 4, and
(c) an optional peptide linker;
   wherein the fusion protein has hyper-cytokine activity, preferably at least 10%, at least 20%, at least 30%, at least 40%, at least 50%, at least 60%, at least 70%, at least 80%, at least 90%, at least 100% or more of the activity of the sIL-11R-IL-11 fusion protein according to SEQ ID NO: 11, when tested in a relevant assay of IL-11 activity.

In a further preferred embodiment of the composition of the first aspect, the hyper-cytokine comprises, consists essentially of or consists of:
(a) a polypeptide having the amino acid sequence according to SEQ ID NO: 5 to 11; or
(b) a polypeptide exhibiting at least 90% sequence identity to a polypeptide according to (a) and having hyper-cytokine activity.

The polypeptide having the amino acid sequence according to SEQ ID NO: 5 consists of the part from P113 to A323 of IL-6R according to SEQ ID NO: 1, a glycine-rich linker sequence of 13 amino acids, and the part from P29 to M212 of IL-6 according to SEQ ID NO: 2 (Fischer et al., 1997). Further sIL-6R and IL-6 fusion proteins having the amino acids sequences according SEQ ID NO: 6, 7 and 8 have been described by Chebath et al. and consist of the region from M1 to V356 of IL-6R according to SEQ ID NO: 1, the region from P29 to M212 of IL-6 according to SEQ ID NO:2, and different linker sequences. The polypeptide according to SEQ ID NO: 6 comprises a 3 amino acid linker sequence (EFM), the polypeptide according to SEQ ID NO: 7 comprises a 13 amino acid linker sequence (EFGAGLVLGGQFM; SEQ ID NO: 12), and the polypeptide according to SEQ ID NO: 8 contains no linker sequence. Additional, fusion proteins have been described in WO 97/32891 and comprise amino acids M1 to A323 of sIL-6R according to SEQ ID NO: 1 and amino acids and P29 to M212 of IL-6 according to SEQ ID NO:2 linked by either a 13 amino acid linker sequence (RGGGGSGGGGSVE, SEQ ID NO: 13) according to SEQ ID NO: 9 or a 18 amino acid linker sequence (RGGGGSGGGGSGGGGSVE; SEQ ID NO: 14) according to SEQ ID NO: 10. The fusion protein having the amino acid sequence according to SEQ ID NO: 11 comprises the region from M1 to Q365 of sIL-11R and the region from A19 to L199 of IL-11. The sIL-6R IL-6 fusion according to SEQ ID NO: 9 is a particularly preferred embodiment and is referred herein as "Hyper-IL-6" or "H6". A preferred expression cassette comprising Hyper-IL-6 and the Neo selectable marker both under the control of the CMV immediate early promoter is provided as SEQ ID NO: 15. This cassette may be comprised in a variety of vectors, preferably viral vectors like retroviral vectors.

The second cell, preferably the allogenic second cell, has a different human leukocyte antigen (HLA) type than the first cell, preferably the allogenic first cell. The HLA system is the name used for the human major histocompatibility complex (MHC). The group of genes encoding this complex resides on chromosome 6, and encodes cell-surface antigen-presenting proteins and many other genes. The major HLA antigens are essential elements in immune function.

Different classes have different functions
(a) class I antigens (A, B & C) - Present peptides from inside the cell (including viral peptides if present), and
(b) class II antigens (DR, DP, & DQ) - Present phagocytosed antigens from outside of the cell to T-lymphocytes

Aside from the genes encoding the 6 major antigens, there are a large number of other genes, many involved in immune function located on the HLA complex. Diversity of HLA in human population is one aspect of disease defence, and, as a result, the chance of two unrelated individuals having identical HLA molecules on all loci is very low. The proteins encoded by HLAs are the proteins on the outer part of body cells that are (effectively) unique to that person. The immune system uses the HLAs to differentiate self cells and non-self cells. Any cell displaying that individuals' HLA type belongs to that individual (and therefore is not an invader). Long before PCR based gene sequencing and gene identification were available, the HLA antigens were recognized as factors interfering with or, occasionally, permitting successful transplantion. Donor organs transplanted into recipients elicit antibodies against the donor's tissues and turning the donor's HLA receptors into antigens of the recipients immune system, hence the name 'human leukocyte antigens'. The types of receptors could be classified based on the antibodies that they induced. These antibodies, particularly to donors who were homozygotes of a particular class II haplotype can be used to identify different receptor types and isoforms. There are two parallel systems of nomenclature that are used to classify HLA. The, first, and oldest system is based on serological (antibody based) recognition. In this system antigens are eventually assigned letters and numbers (e.g. HLA-B27 or, shortened, B27). A parallel system has been developed that allowed more refined definition of alleles, in this system a "HLA" is used in conjunction with a letter, an asterisk (*), and a four or more digit number (e.g. HLA-B*0801, A*68011, A*240201N N=Null) to designate a specific allele at a given HLA locus. HLA loci can be further classified into MHC class I and MHC class II (or rarely, D locus). This classification is based on sequence information from the respective HLA loci. Accordingly, the skilled person is well aware how to determine, whether two groups of cells have the same or a different HLA type. Preferably, the first and the second cell line have a different HLA type based on the antibody type classification system.

The one or more first cells and/or the one and more second cells is a tumour cell. Preferably, the tumour cell is selected independently for each cell from the group consisting of a melanoma cell, a renal carcinoma cell, a prostate cancer cell, a colon cancer cell, a lung cancer cell, a pancreas cancer cell, a liver cancer cell, a brain cancer cell, a head and neck cancer cell, and a sarcoma cell. Preferably, the first and the second cells are selected from the same tumour cell type but either from different tumours within an individual or from two different individuals.

In one embodiment of the first aspect, the first cells, which are modified to express a hypercytokine are the human (Homo sapiens) melanoma derived cells Mich1, deposited on April 24, 2007 under accession number DSM ACC2837 with the "Deutsche Sammlung von Mikroorganismen und Zellkulturen" (DSMZ), Inhoffenstr. 7 B, 38124 Braunschweig, Germany and/or the second cells, which are modified to express a hypercytokine are the human (Homo sapiens) melanoma derived cells Mich2, deposited on April 24, 2007 under accession number DSM ACC2838 with the DSMZ. Mich1 and Mich2 originate from different patients.

In a preferred embodiment, the first cells are Mich1-H6, deposited on April 24, 2007 under accession number DSM ACC2839 with the DSMZ. In a preferred embodiment, the second cells are Mich2-H6, deposited on April 24, 2007 under accession number DSM ACC2840 with the DSMZ. Mich1-H6 and Mich2-H6 have respectively been derived from infection of Mich1 and Mich2 with a retrovirus comprising the expression cassette according to SEQ ID NO: 15 and expressing Hyper-IL-6 according to SEQ ID NO: 9 under the control of the CMV promoter.

The *in vivo* anti tumour effect exerted by compositions comprising first and second cells, in particular tumour cells, modified to express a hypercytokine can be further enhanced, if the first and/or second cells are engineered to comprise at least one further polynucleotide encoding an antigen, preferably a tumour antigen, a cytokine, in particular GM-CSF, IL-2, IL-6, IL-7, IL-11, IL-15, IL-21, anti-TGF, EPO, interferon, in particular INF-α, LIF, OSM, CNTF, CT-1 or a hypercytokine different from the first hypercytokine comprised in the respective cell. The engineering is preferentially achieved by using a vector, in particular one of the expression vectors indicated above with respect to hypercytokines and the subsequent or simultaneous introduction of this(ese) vector(s) into the first and/or second cells to be modified. The one or more additional polynucleotide can be comprised in a separate vector or can be comprised within the same vector as the hypercytokine encoding polynucleotide. It is preferred that the host cells simultaneously express both the hypercytokine and the at least one further protein encoded by the at least one further polynucleotide.

The term "tumour antigen" comprises all substances, which elicit an immune response against a tumour. Particular suitable substances are proteins or protein fragments which are enriched in a tumour cell in comparison to a healthy cell. These substances are preferably present within and/or are accessible on the outside of the tumour cell. If the tumour antigen is only present within a tumour cell, it will still be accessible for the immune system, since the antigen or fragments thereof will be presented by the MHC system at the surface of the cell. In a preferred aspect the tumour antigen is almost exclusively or exclusively present on and/or in the tumour cell and not in a healthy cell of the same cell type.

Suitable tumour antigens can be identified, for example, by analyzing the differential expression of proteins between tumour and healthy cells of the same cell type using a microarray-based approach (Russo et al., Oncogene. 2003, 22:6497-507), by PCR- or microarray-based screening for tumour specific mutated cellular genes (Heller, Annu. Rev. Biomed. Eng. 2002, 4:129-53) or by serological identification of antigens by recombinant expression cloning (SEREX; Tureci et al., Mol Med Today. 1997, 3:342-349). The skilled artisan is aware of a large number of substances which are preferentially or exclusively present on and/or in a tumour cell, which include for example, oncogenes like, for example truncated epidermal growth factor, folate binding protein, melanoferrin, carcinoembryonic antigen, prostate-specific membrane antigen, HER2-neu.

Not all of the substances that are preferentially or exclusively present in and/or on a tumour cell will elicit a strong immune response, therefore, it is preferred that tumour antigens are selected to be expressed in the first and/or second cells of the composition of the invention, which elicit a strong immune response. Antigens eliciting a strong immune response will induce at least 1%, preferably at least 5%, more preferably at least 10% and most preferably at least 15% IFNγ-producing CD8+ T or CD4+ T cells isolated from mice previously immunized with the antigen, upon challenge with the antigen and/or will induce preferably at least 5%, and most preferably at least 15% of B-cells cells isolated from mice previously immunized with the antigen, upon challenge with the antigen to proliferate. Antigens fulfilling these criterions are candidates to be expressed in the cancer vaccine composition of the present invention.

In a particular preferred embodiment the tumour antigen is selected from the group consisting of T-cell-defined cancer-associated antigens belonging to unique gene products of mutated or recombined cellular genes, in particular cyclin-dependent kinases (e.g. CDC2, CDK2, CDK4), p15^{Ink4b}, p53, AFP, β-catenin, caspase 8, p53, p21^{Ras} mutations, Bcr-abl fusion product, MUM-1 MUM-2, MUM-3, ELF2M, HSP70-2M, HST-2, KIAA0205, RAGE, myosin/m, 707-AP, CDC27/m, ETV6/AML, TEL/Aml1, Dekcain, LDLR/FUT, Pml-RARα, TEL/AMLI; Cancer-testis (CT) antigens, in particular NY-ESO-1, members of the MAGE-family (MAGE-A1, MAGE-A2, MAGE-A3, MAGE-A4, MAGE-A6, MAGE-10, MAGE-12), BAGE, DAM-6, DAM-10, members of the GAGE-family (GAGE-1, GAGE-2, GAGE-3, GAGE-4, GAGE-5, GAGE-6, GAGE-7B, GAGE-8), NY-ESO-1, NA-88A, CAG-3, RCC-associated antigen G250; Tumour virus antigens, in particular human papilloma virus (HPV)-derived E6 or E7 oncoproteins, Epstein Barr virus EBNA2-6, LMP-1, LMP-2; overexpressed or tissue-specific differentiation antigens, in particular gp77, gp100, MART-1/Melan-A, p53, tyrosinase, tyrosinase-related protein (TRP-1 and TPR-2), PSA, PSM, MC1R; widely expressed antigens, in particular ART4, CAMEL, CEA, CypB, HER2/neu, hTERT, hTRT, iCE, Mucl, Muc2, PRAME RU1, RU2, SART-1, SART-2, SART-3, and WT1; and fragments and derivatives thereof. Particular preferred tumour antigens are antigens derived from the tyrosinase-related protein.

When the composition of the present invention is administered to a patient it is administered to elicit an immune response both against the first and/or second cells and any tumour cells, which share epitopes and/or tumour antigens with the first and/or second cells. It is, thus, expected that the cells of the composition will only survive for a limited time within the recipient of the compositions of the present invention and are then cleared from the organism of the recipient by the immune system of the recipient. Nevertheless, it is preferred for safety reasons that the proliferation of the first, preferably allogenic and/or the second, preferably allogenic cells has been inhibited prior to the administration of these cells to a patient. The term "inhibition" comprises both the slowing down of the proliferation rate and the complete cessation of proliferation. The skilled person is aware of a larger number of chemical and physical methods, which affect the growth rate of cells, these include without limitation radiation, e.g. γ-irradiation or cross-linking, e.g. psoralen or aldehyde. The level of inhibition, however, should preferably be such, that transcription and translation of the transgenes introduced into the first and second cells is not completely shut down, i.e. the transgenes should be expressed at a level of at least 5%, preferably at least 10%, at least 20%, at least 30%, at least 40%, at least 50%, at least 60%, at least 70%, at least 80%, at least 90%, at least 100% or more of the first and/or second cells prior to inhibition. Preferably, the cells are capable to continue to go through 1 to 5, i.e. 1, 2, 3, 4, or 5, replication cycles after the chemical or physical method for inhibition of proliferation is administered.

The anti-tumour effect of the composition of the present invention can be further enhanced, if one or more additional cells, which may be autologous or allogenic, which are different from the first and/or the second cells are also included in the composition. Preferably these cells originate from a further individual, preferably having a HLA type different from the HLA types of the first and/or second cell types. Again it is preferred that these cells are tumour cells, preferably from the same tumour type as the first and/or second cells. For the reasons outlined above it is also preferred that the proliferation of the one or more additional, preferable allogenic cells has been inhibited, preferably as outlined above.

It is particularly preferred that the one or more cells of the one or more additional allogenic cells have been modified to express a cytokine, a cytokine receptor, a hypercytokine and/or a tumour antigene. Preferably, the cytokine is selected from the group consisting of GM-CSF, IL-2, IL-6, IL-7, IL-11, IL-15, IL-21, anti-TGF, EPO, interferon, in particular INF-α, LIF, OSM, CNTF, CT-1 and the cytokine receptors or soluble parts thereof are those receptors corresponding to the indicated cytokines. Preferably, the hypercytokine is selected from the group consisting of hyper-IL-6, e.g. according to SEQ ID NO: 5, 6, 7, 8, 9 or 10, IL-2, hyper-IL-11, e.g. according to SEQ ID NO: 11, hyper CNTF, and hyper-OSM.

As it is intended that the composition of the present invention elicits an immune response the composition may further comprise adjuvants, which are commonly used in vaccines to enhance the immunizing effect. Preferred adjuvants are selected from the group consisting of unmethylated DNA, in particular unmethylated DNA comprising CpG dinucleotides (CpG motif), in particular CpG ODN with phosphorothioate (PTO) backbone (CpG PTO ODN) or phosphodiester (PO) backbone (CpG PO ODN); gel-like precipitates of aluminum hydroxide (alum); bacterial products from the outer membrane of Gram-negative bacteria, in particular monophosphoryl lipid A (MPLA), lipopolysaccharides (LPS), muramyl dipeptides and derivatives thereof; synthetic lipopeptide derivatives, in particular Pam₃Cys; lipoarabinomannan; peptidoglycan; zymosan; heat shock proteins (HSP), in particular HSP 70; dsRNA and synthetic derivatives thereof, in particular Poly I:poly C; polycationic peptides, in particular poly-L-arginine; taxol; fibronectin; flagellin; imidazoquinoline; cytokines with adjuvant activity, in particular GM-CSF, interleukin- (IL-)2, IL-6, IL-7, IL-18, type I and II, interferons, in particular interferon-gamma, TNF-alpha; oil in water emulsions, in particular MF59 consisting of squalene; Tween 80 and Span 85 (sorbitan-trioleate) and QS-21, a more highly purified derivative of Quil A, non-ionic block polymers, in particular Poloxamer 401, saponins and derivatives thereof, in particular the immunostimulatory fragments from saponins; polyphosphazene; N-(2-Deoxy-2-L-leucylamino-β-D-glucopyranosyl)-N-octadecyldodecanoylamide hydroacetate (BAY R1005), 25-dihydroxyvitamin D3 (calcitriol); DHEA; murametide [MDP(Gln)-OMe]; murapalmitine; polymers of lactic and/or glycolic acid; polymethyl methacrylate; sorbitan trioleate; squalane; stearyl tyrosine; squalene; theramide, synthetic oligopeptides, in particular MHCII-presented peptides. Particular preferred adjuvants, which can be comprised in the compositions of the present invention are selected from the group unmethylated DNA, in particular unmethylated DNA comprising CpG dinucleotides (CpG motif), in particular CpG ODN with phosphorothioate (PTO) backbone (CpG PTO ODN) or phosphodiester (PO) backbone (CpG PO ODN) and synthetic lipopeptide derivatives, in particular Pam₃Cys.

In a further aspect the present invention concerns a composition of the present invention for use in medicine.

In a further aspect the present invention concerns a pharmaceutical composition comprising a composition of the invention additionally comprising pharmaceutically acceptable diluents, carriers, excipients, fillers, binders, lubricants, glidants, disintegrants, adsorbents, and/or preservatives. Preferably the pharmaceutical composition is formulated for parenteral use, preferably in the form of a sterile aqueous solution which may contain other substances, for example, enough salts or glucose to make the solution isotonic with blood. The aqueous solutions should be suitably buffered (preferably to a pH of from 3 to 9), if necessary. A particularly preferred aqueous solution is phosphate buffered saline (PBS).

Preferably a unit dose of a composition of the present invention comprises between at least 1 x 10⁵ and 1 x 10⁹ cells of first cells, preferably at least 2 x 10⁵, 3 x 10⁵, 4 x 10⁵, 5 x 10⁵, 6 x 10⁵ , 7 x 10⁵, 8 x 10⁵, 9 x 10⁵, 1 x 10⁶, 2 x 10⁶, 3 x 10⁶, 4 x 10⁶, 5 x 10⁶, 6 x 10⁶, 7 x 10⁶, 8 x 10⁶, 9 x 10⁶, 1 x 10⁷, 2 x 10⁷, 3 x 10⁷, 4 x 10⁷, 5 x 10⁷, 6 x 10⁷, 7 x 10⁷, 8 x 10⁷, 9 x 10⁷, and 1 x 10⁸. A particular preferred unit dose of a composition of the present invention comprises between 1 x 10⁷ to 1 x 10⁸ first cells, preferably 2.5 x 1 x 10⁷. Additionally, the unit dose of a composition of the present invention comprises between at least 1 x 10⁵ and 1 x 10⁹ cells of second cells, preferably at least 2 x 10⁵, 3 x 10⁵, 4 x 10⁵, 5 x 10⁵, 6 x 10⁵, 7 x 10⁵, 8 x 10⁵, 9 x 10⁵, 1 x 10⁶, 2 x 10⁶, 3 x 10⁶, 4 x 10⁶, 5 x 10⁶, 6 x 10⁶, 7 x 10⁶, 8 x 10⁶, 9 x 10⁶, 1 x 10⁷, 2 x 10⁷, 3 x 10⁷, 4 x 10⁷, 5 x 10⁷, 6 x 10⁷ , 7 x 10⁷, 8 x 10⁷, 9 x 10⁷, and 1 x 10⁸. A particular preferred unit dose of a composition of the present invention comprises between 1 x 10⁷ to 1 x 10⁸ second cells, preferably 2.5 x 1 x 10⁷. Preferably, the composition comprises about the same number of first cells and second cells for a total of 2 x 10⁵ to 2 x 10⁸ cells per unit dose. A particular preferred unit dose of a composition of the present, invention comprises between 2 x 10⁷ to 2 x 10⁸ first and second cells, preferably 5 x 10⁷. The total volume of the unit dose is preferably between 0.5 to 20 ml, preferably, 1 to 5 ml, e.g. 1, 2, 3, 4, or 5 ml.

In a further aspect the present invention relates to the composition, of the present invention or the pharmaceutical composition of the present invention for use in the treatment or prevention of cancer. Preferred cancers treatable or preventable with a composition according to the present invention are selected from the group consisting of cancer of the gastrointestinal or colorectal tract, liver, pancreas, kidney, bladder, prostate, endometrium, head and neck cancer, ovary, testes, prostate, skin, eye, melanoma, dysplastic oral mucosa, invasive oral cancer, small cell and non-small cells lung cancer, hormone-dependent breast cancer, hormone independent breast cancer, transitional and squamous cell cancer, neurological malignancy, including neuroblastoma, glioma, astrocytoma, osteosarcoma, soft tissue sarcoma, hemangioma, endocrinological tumour, hematologic neoplasia including leukemia, lymphoma, and other myeloproliferative and lymphoproliferative diseases, carcinoma in situ, hyperplastic lesion, adenoma, and fibroma. Particular preferred is the treatment or prevention of melanoma, pancreatic and renal cancer.

In a further aspect the present invention relates to the use of a composition of the present invention for the preparation of a pharmaceutical composition for the treatment or prevention of cancer.

The compositions of the invention can be used in the treatment and/or prevention of a wide variety of different cancers, however, preferred cancers treatable or preventable according to the present invention are selected from the group consisting of cancer of the gastrointestinal or colorectal tract, liver, pancreas, kidney, bladder, prostate, endometrium, head and neck cancer, ovary, testes, prostate, skin, eye, melanoma, dysplastic oral mucosa, invasive oral cancer, small cell and non-small cell lung cancer, hormone-dependent breast cancer, hormone independent breast cancer, transitional and squamous cell cancer, neurological malignancy, including neuroblastoma, glioma, astrocytoma, osteosarcoma, soft tissue sarcoma, hemangioma, endocrinological tumour, hematologic neoplasia including leukemia, lymphoma, and other myeloproliferative and lymphoproliferative diseases, carcinoma in situ, hyperplastic lesion, adenoma, and fibroma. Particularly preferred is the treatment or prevention of melanoma, pancreatic and renal cancer. In particular in the context of the treatment and/or prevention of cancer it is envisionable that patients are immunized with a "cancer vaccine" prior to the development of any symptoms of a disease, i.e. receive a protective immunization, or after they have developed symptoms of the disease, i.e. receive a therapeutic vaccination.

The expression of at least one further cytokine, in particular GM-CSF, by the first and/or second cells expressing a hypercytokine, preferably Hyper-IL-6 can provide in the context of certain tumours, in particular melanoma and renal cancer an even stronger *in vivo* anti-tumour response than cells expressing only the hypercytokine. Therefore, in a preferred use the first and/or the second cells expressing hypercytokine are modified to express at least one further cytokine are used for the production of a medicament to prevent or treat a proliferative disease.

It is particularly preferred in this context when the first and the second cells are from the same type of tissue, preferably tumour tissue but have a partially or completely different HLA type than the first cell and/or the second cell.

### EXAMPLES

In the following, the invention is explained in more detail by non-limiting examples:

### Example 1: Hyper-IL-6 (H6) augments T-cell proliferative response in allogenic mixed tumour-lymphocyte reaction (AMTLR)

Irradiated tumour cells were mixed with unprimed, allogenic lymphocytes in the presence or absence of IL-6 (1 ng/ml) or purified H6 (1 ng/ml). After three days, T-cells were assayed for proliferation by ³H-thymidine incorporation, determined as counts per minute (cpm). The results of this experiment are shown in Fig. 1.

Columns 1 to 3 show the results of spontaneous T-cell proliferation, i.e. in the absence of tumour cells. Apparently, spontaneous T-cell proliferation does not occur to a significant extent (columns 1 to 3), irrespective whether IL-6 (column 2) or H6 (column 3) are added to the mixture.

Columns 4 to 6 show the results of T-cell proliferation in response to allogenic tumour cells. In the presence of allogenic tumour cells, the T-cells show very strong proliferation (column 4). The addition of IL-6 has no apparent effect on the proliferation of T-cells (column 5). The addition of H6 (column 6) leads to an almost two-fold increase in T-cell proliferation. Thus, H6 strongly enhances the T-cell proliferative response in an allogenic mixed tumour-lymphocyte reaction (AMTLR).

### Example 2: T-cell proliferation in allogenic mixed tumour-lymphocyte reaction is dependent on IL-2.

Irradiated tumour cells were mixed with unprimed, allogenic lymphocytes in the presence or absence of IL-6 (1 ng/ml), purified H6 (1 ng/ml) and anti-IL-2 antibody (1 µg/ml). After three days T-cells were assayed for proliferation by ³H-thymidine incorporation, determined as cpm. The results of this experiment are shown in Fig. 2.

Columns 1 to 3 of Fig. 2 show the results of T-cell proliferation in response to allogenic tumour cells in the absence of anti-IL-2 antibody. The results from this experiment are almost identical to the results presented in Fig.1, columns 4 to 6. As shown in Example 1, T-cells show a very strong proliferation in the presence of allogenic tumour cells (column 1 of Fig. 2). The addition of IL-6 has no apparent effect on the proliferation of T-cells (column 2 of Fig. 2). The addition of H6 leads to an almost two-fold increase in T-cell proliferation (column 3 of Fig. 2).

Columns 4 to 6 of Fig. 2 show the results of T-cell proliferation in response to allogenic tumour cells in the presence of anti-IL-2 antibody, which neutralizes the effect of IL-2. The addition of the anti-IL-2 antibody greatly reduces T-cell proliferation (columns 4 to 6). The presence of IL-6 (column 5) or H6 (column 6) has no apparent effect on T-cell proliferation when anti-IL-2 antibody is present. Thus, the T-cell proliferation in the allogenic mixed tumour-lymphocyte reaction (AMTLR) is dependent on IL-2.

### Example 3: Hyper-IL-6 augments IL2 and IFN-γ production by T-cells in allogenic mixed tumour-lymphocytic reaction

This example describes the evaluation of the immunostimulatory potential of Hyper-IL-6 (H6) in a mixed allogenic tumour/lymphocyte reaction. Results obtained indicate that Hyper-IL-6 increases immunostimulatory potential of allogenic melanoma cells. Moreover, Hyper-IL-6 is not only more potent than IL-6 but also displays qualitatively different biological activity. In contrast to native IL-6 which is a known Th2 inducer, Hyper-IL-6 appears to reduce IL-10 expression while increasing IFN-γ and IL-2 production by peripheral blood lymphocytes (PBLC) which is characteristic of a Th1 response.

Test Articles: A375 melanoma cells and their derivative A375-H6 cells; PBLC isolated from a healthy volunteer.

Media, components and equipment: FBS (GIBCO/Invitrogen), PBS (GIBCO/Invitrogen), DMEM (GIBCO/Invitrogen), Trypsin EDTA (GIBCO/Invitrogen), Tissue culture flask 25cm² (Sarstedt), 24 well plate (Nunc), Lymphocyte separation medium (ICN), BD Cytometric Bead Array (CBA) Human Th1/Th2 Cytokine Kit-II (Becton Dickinson), IL-6 (Pharmingen), Flow cytometer (Becton Dickinson), FACSAria™ (Becton Dickinson).

### Methods:

PBLC drawn from a healthy volunteer were separated from whole blood by centrifugation over lymphocyte separation medium. Cells were washed twice in PBS and counted by standard procedures in a haemocytometer. Lymphocytes were re-suspended at 2 x 10⁶ cells per ml in DMEM culture medium supplemented with 2% FBS. Tumour cells were trypsinized, washed twice in PBS and re-suspended at 2 x 10⁶ cells per ml in DMEM medium supplemented with 2% FBS. 0.5 ml of lymphocyte suspension was mixed with 0.5 ml of tumour cells and seeded on a 24 well plate to give 1 ml of mixed cell culture.

### Experimental settings:

0.5 ml of lymphocyte suspension + 0.5 ml of culture medium (control)
0.5 ml of lymphocyte suspension + 0.5 ml of culture medium + 10 ng of IL-6
0.5 ml of lymphocyte suspension + 0.5 ml of A375 tumour cells suspension
0.5 ml of lymphocyte suspension + 0.5 ml of A375 cells suspension + 10 ng of IL-6
0.5 ml of lymphocyte suspension + 0.5 ml of A375-H6 cells suspension
0.5 ml of A375 tumour cells suspension + 0.5 ml of culture medium
0.5 ml of A375-H6 tumour cells suspension + 0.5 ml of culture medium

The mixed cells were cultured for three days in a humidified cell incubator at 37°C, 5% CO₂/ 95% air. After 3 days cell-free supernatant was collected and analyzed for cytokine content. Collected supernatants were properly marked and immediately frozen at -20°C until analysis. Cytokine content was determined by CBA within one month according to the instruction provided in the CBA manual.

The results of this example are summarized in Table 1 below. They show that the A375 tumour cells stimulate allogenic T-cells to produce IL-2, IL-6, IL-10 and IFN-γ. The presence of hyper-IL6 but not IL-6 significantly augmented IL-2 and IFN-γ production in T-cells and at the same time reduced IL-10 secretion.

**Table 1. Cytokine production by allogenic tumour reactive T-cells**

| | cytokines pg/ml | | | | | |
|---|---|---|---|---|---|---|
| | IL-2 | IL-4 | IL-6 | IL-10 | TNF-α | IFN-γ |
| A375 | 0 | 0 | 48 | 0 | 0 | 0 |
| A375-H6 | 0 | 0 | >5000 | 46 | 0 | 0 |
| PBLC | 3 | 0 | 583 | 11 | 0 | 0 |
| PBLC + IL-6 | 2 0 | | 283 | 7 | 0 | 0 |
| A375 + PBLC | 92 | 0 | >5000 | 113 | 0 | 19 |
| A375 + IL-6 + PBLC | 96 | 0 | >5000 | 140 | 0 | 19 |
| A375-H6 + PBLC | 348 | 0 | >5000 | 78 | 0 | 106 |

From the above results it is apparent that hyper-IL-6 is not only more potent then IL-6 but also displays a qualitatively different biological activity. In contrast to native IL-6 which is a known Th2 inducer, hyper-IL-6 appears to reduce IL-10 expression while increasing IFN-γ and IL-2 production characteristic for Th1 response. This type of T-helper response (i.e. Th1) is of primary importance during cytotoxic T-cell induction and development and is therefore a desirable response in anti-tumour vaccines.

Without wishing to be bound by a single explanation, the inventors assume that the apparent lack of IL-6 activity added in excess into culture media can be explained as follows: From the above experiments it appears that allogenic tumour cells on their own induce very potent IL-6 production in reacting T-cells. This production reaches very high levels and most likely saturates all IL-6 specific receptors. As a result, addition of IL-6 into the culture system does not have any influence on T-cell behavior. On the other hand hyper-IL6 does not need any free IL-6 receptor, instead it binds to a common gp130 receptor subunit. Moreover, since hyper-IL6 does not need a specific IL-6 receptor it may operate on different cell populations compared to IL-6. The observed effects may be due to extra stimuli of IL-6 responding T-cells or independent T-cell stimulation which are negative for IL-6 receptor.

From the results presented in this Example it is apparent that under *in vitro* conditions hyper-IL-6 significantly increases stimulatory potential of human allogenic melanoma cells by shifting the immune response towards a Th1 type i.e. a cellular response.

### Example 4: Synthesis of cytokines, growth factors and vascular factors by melanoma cells and H6-modified melanoma cells

The aim of this example was to assess the synthesis of selected cytokines, growth and vascular factors by melanoma cells which can be used as components in a vaccine. A further aim was to evaluate the effect of the H6 gene modification on the synthesis of above factors by said melanoma cells. Specific aims included (i) analysis of secretion of soluble factors such as IL-2, IL-4, IL-6, IL-8, IL-10, IL-12, INFγ, GM-CSF, RANTES and VEGF by melanoma cells Mich1 and Mich2; (ii) analysis of secretion of above factors by Mich1-H6 and Mich2-H6 cells (H6-modified melanoma cells); (iii) comparison of secretion pattern of H6-modified and parental Mich1 and Mich2 cells.

Test Articles: Mich1 cells, Mich2 cells, Mich1-H6 cells and Mich2-H6 cells were thawed and cultured for two days and passaged for the next 3 days. Then cells were trypsinized and frozen. These cells (passage 1 - P1) were used in the experiment.

**Table 2. Deposit numbers of cell lines used**

| Name of cell line | International Depository Authority | Date of Deposit | Accession number given by the International Depository Authority |
|---|---|---|---|
| Mich1 | DSMZ¹ | 24 April 2007 | DSM ACC2837 |
| Mich2 | DSMZ | 24 April 2007 | DSM ACC2838 |
| Mich1-H6 | DSMZ | 24 April 2007 | DSM ACC2839 |
| Mich2-H6 | DSMZ | 24 April 2007 | DSM ACC2840 |

| | | | |
|---|---|---|---|
| DSMZ - Deutsche Sammlung von Mikroorganismen und Zellkulturen GmbH, Inhoffenstr. 7 B, D-38124 Braunschweig, Germany | | | |

Methodology: The cell lines studied were thawed and seeded in culture flasks. Cells were cultured until confluency and then maintained in serum (FBS) free medium (DMEM) for 48 hr in 5% CO₂ humidified atmosphere. Then the media were collected and analyzed for the above-listed factors using multiplex particle-based immunoassay with FC readout.

### Secretion of analyzed factors by the four cell lines studied

Results of the amounts of the cytokines and growth factors secreted into the culture media by Mich1 Mich2 and Mich1-H6, Mich2-H6 cells are summarized in Table 3.

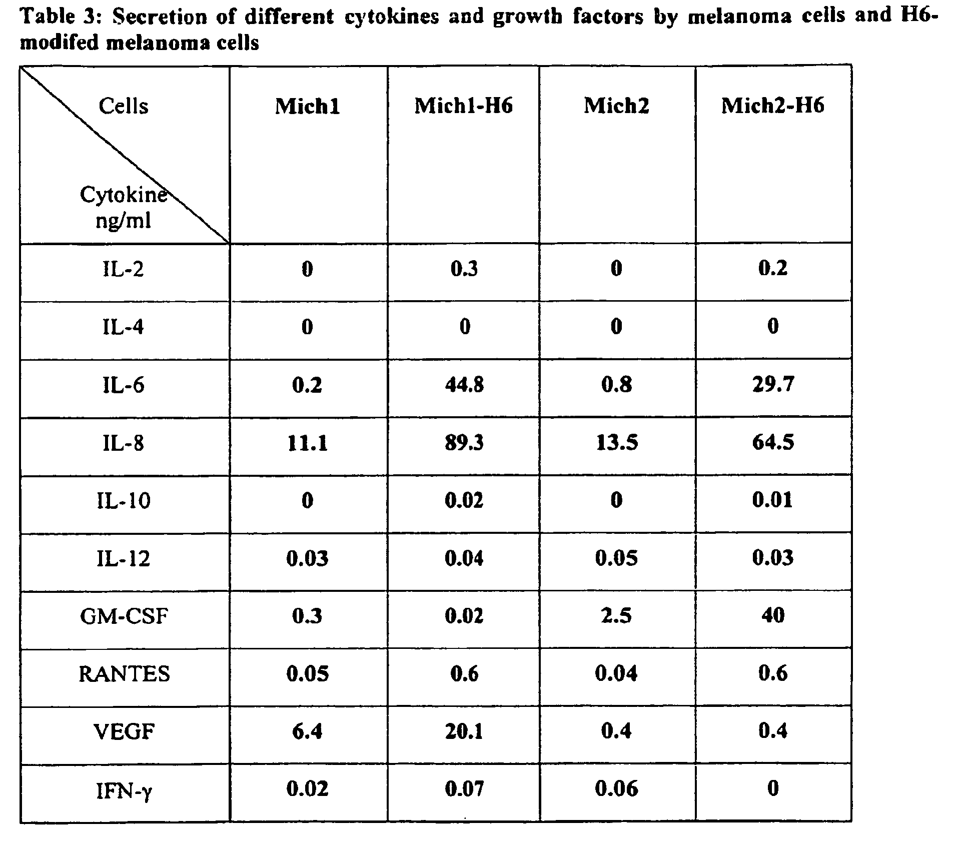

Mich1 cells secreted low levels of IL-12, RANTES, and INF-γ; and moderate levels of IL-6 and GM-CSF; and relatively high levels of IL-8 and VEGF. IL-2, IL-4 and IL-10 were either not secreted or secreted at extremely low levels which were below the detection limit of the assay.

Mich2 cells secreted low levels of IL-12, RANTES and INF-γ; moderate levels of IL-6 and VEGF; and high levels of IL-8 and GM-CSF. IL-2, IL-4 and IL-10 were not detectable in the assay.

Mich1-H6 cells secreted low levels of IL-10, IL-12, GM-CSF and INF-γ; moderate levels of IL-2 and RANTES; high levels of IL-6 and VEGF; and very high levels of IL-8. IL-4 was not detectable in the assay.

Mich2-H6 cells secreted low levels of IL-10 and IL-12; moderate levels of IL-2, RANTES and VEGF; high levels of IL-6 and very high levels of IL-8 and GM-CSF. IL-4 and INF-γ were not detectable in the assay.

### Comparison of the secretion pattern of studied factors by Mich1 and Mich2 cells

A comparison of the secretion pattern of the studied factors by parental cell lines Mich1 and Mich2 demonstrated significant qualitative similarities (see Table 3). Cells of both lines secreted IL-6, IL-8, IL-12, GM-CSF, RANTES, VEGF and INF-γ, but did not secrete IL-2, IL-4 and IL-10. However, some quantitative differences in GM-CSF and VEGF secretion between both lines were observed.

### Comparison of the secretion pattern of studied factors by Mich1-H6 and Mich2-H6 cells

A comparison of the secretion pattern of studied factors by the modified cell lines Mich1-H6 and Mich2-H6 in general revealed qualitatively similar secretion patterns (see Table 3). Cells of both lines secreted IL-2, IL-6, IL-8, IL-10, IL-12, GM-CSF, RANTES and VEGF. Mich1-H6 cells secreted INF-γ, while Mich2-H6 did not. None secreted IL-4. Significant quantitative differences were seen for GM-CSF and VEGF secretion between both lines (3 and 2 orders of magnitude, respectively).

### Effect of the H6 modification on the secretion pattern of studied factors by melanoma cell lines

Mich1 cells: Significant qualitative (IL-2, IL-10) and quantitative differences between Mich1 and Mich1-H6 cells are observed. Except for GM-CSF which was decreased, expression of all other factors studied was significantly increased in H6 modified cells.

Mich2 cells: Significant qualitative (IL-2, IL-10, INF-γ) and quantitative differences between Mich2 and Mich2-H6 cells are observed. Except for IL-12 and VEGF which were at the same level, expression of all factors studied was significantly increased. In contrast, Mich2-H6 cells did not express INF-γ.

### Summary of results from Example 4

Mich1 and Mich2 cells secreted 7 out of 10 factors studied. H6 modification resulted in the induction of 2 additional proteins (IL-2 and IL-10) and increased production of most of the other factors studied. Some factors such as IL-8, VEGF or GM-CSF were secreted by modified cells in the tenths of nanograms. IL-8 is a very strong chemoattractant increasing recruitment of immune cells into the vaccine injection site. GM-CSF is a major stimulator of dendritic cell maturation, hence inducing antigen presentation. VEGF is a signaling protein involved in vasculogenesis and angiogenesis. It is also capable of stimulating monocyte/macrophage migration. IL-2, IL-12 and INF-γ display immunomodulatory functions on T cells. Modified cells also secreted IL-10 which is considered to be an immunoinhibitory factor. However, experimental studies demonstrated that murine melanoma cells modified with IL-10 cDNA elicited specific anti-melanoma immune responses indicating that in such setting IL-10 provides a stimulatory signal for T lymphocytes. Moreover, modified cells secreted significant quantities of IL-6. However, additional identification studies are necessary since anti-IL-6 antibodies may react with H6 protein. Accordingly, high IL-6 levels detected by the employed method in the culture medium may reflect secretion of the transgenic H6 protein but not necessarily the native IL-6 protein by vaccine cells.

### Example 5: Cytokine production of a mixture of two H6-modified melanoma cell lines in an AMTLR

The aim of this example included the analysis of the effect of a mixture of two melanoma cell lines (Mich1-H6 and Mich2-H6) on cytokine production by PBLC isolated from various healthy individuals with different HLA haplotypes. Moreover, the effect of a mixture of two cell lines was compared with each line used alone. Cytokine production was assessed by measurement of cytokine content in culture medium by CBA (Cytometry Bead Assay).

Cells: Mich1-H6 cells and Mich2-H6 cells were obtained from the same source as described in Example 4 and were irradiated; PBLC were isolated from 4 healthy volunteers (3 males and 1 female).

Media, Components and Equipment: DMEM (GIBCO/Invitrogen); FBS (GIBCO/ Invitrogen); PBS (GIBCO/Invitrogen); Lymphocytes Separation Medium (ICN); ³H-thymidine (Amersham Biosciences); Trypsin EDTA (GIBCO/Invitrogen); 96 well plate (Sarstedt); 75 ml tissue culture flasks (Coming); BD Cytometric Bead Array (CBA) Human Th1/Th2 Cytokine Kit-II (BD Biosciences); FACS Aria^{™} (BD Biosciences); Scintillation Counter; Phase-contrast microscope (Olympus); CO₂ Incubator (Sanyo); Centrifuge (Sorvall).

Cell preparation: Cells were cultured per standard procedures in the lab under GMP-Like conditions by a qualified research worker.

Mich1-H6 and Mich2-H6 cells were plated separately into culture flasks in DMEM culture medium supplemented with 10% FBS at a seeding density of approximately 1.67x10⁴ cells per cm². The cells were grown in culture until confluency (3-5 days) and were then trypsinized, washed twice with PBS, re-suspended at 2x10⁶ cells per ml in DMEM medium supplemented with 2% FBS for cytokine production record, then irradiated at 80Gy (⁶⁰Co).

PBLCs were separated from the whole blood collected from 4 healthy individuals (coded: A, D, M and N) by centrifugation over lymphocyte separation medium. Cells were washed twice with PBS and counted by standard procedures in a haemocytometer. PBLCs were then re-suspended at 5x10⁵ cells per ml in DMEM culture medium supplemented with 2% FBS for cytokine production record.

### Set-up of cytokine production analysis

The test was performed on one 96 well plate for the IL-2, IL-4, IL-6, IL-10, TNF-α and IFN-γ cytokine production panel.

The cell suspension was transferred into the 96 well plate in a volume of 100µl per each cell line in one row for each of four individuals. Into each row the cells were transferred in concentration of 2 x 10⁵ cells per well. Each cell line was reduplicated in four variant columns (four samples for each cell line). There were no melanoma cells added into the last but one 16 wells (4 per each of four individuals), i.e. in Row 5, with these wells acting as a negative control (Control -) for spontaneous PBLC proliferation (the control wells contained DMEM + 2% FBS in quantity of 100µl and PBLC in concentration of 5x10⁴ cells per well). There were no PBLCs added into the last 12 wells, i.e. in Row 6, with these wells acting as a positive control (Control +) for spontaneous melanoma cells proliferation (the positive control will contain DMEM +2% FBS in quantity of 100µl and melanoma cells in concentration of 2 x 10⁵ cells per well for Mich1-H6, Mich2-H6 and mixture of both cell lines, respectively).

Then PBLCs were added to each well including the negative control wells (but except positive control wells) in a volume of 100µl and at concentration of 0.5 x 10⁵ cells per well. The total volume in each well was 200µl (i.e. 100µl of PBLC plus 100µl of melanoma cells solutions).

**Table 4: Arrangement of samples and controls**

| **Cell Component** | **Mich1-H6** | **Mich2-H6** | **Mich1-H6 + Mich2-H6** | **Control** |
|---|---|---|---|---|
| Row 1 | 2x10⁵ | 2x10⁵ | 1x10⁵ each | - |
| Row 2 | 2x10⁵ | 2x10⁵ | 1x10⁵ each | - |
| Row 3 | 2x10⁵ | 2x10⁵ | 1x10⁵ each | - |
| Row 4 | 2x10⁵ | 2x10⁵ | 1x10⁵ each | - |
| Control - | 5x10⁴ | 5x10⁴ | 5x10⁴ | 5x10⁴ |
| Control + | 2x10⁵ | 2x10⁵ | 1x10⁵ each | - |

The mixed cells were co-cultured for 3 days for cytokine production analysis, as it is optimal period for incubation, in a humidified incubator at 37°C in 5% CO₂/95% air atmosphere.

After the predefined period of three days cell free supernatants from the plate were collected, properly marked and immediately frozen at -20°C until further analysis. Cytokines accumulated in the medium were measured using BDTM Biosciences Cytometric Bead Array (CBA) Human Th1/Th2 Cytokine Kit-II Assay within one month according to the instruction provided in the CBA manual.

### Cytokine secretion

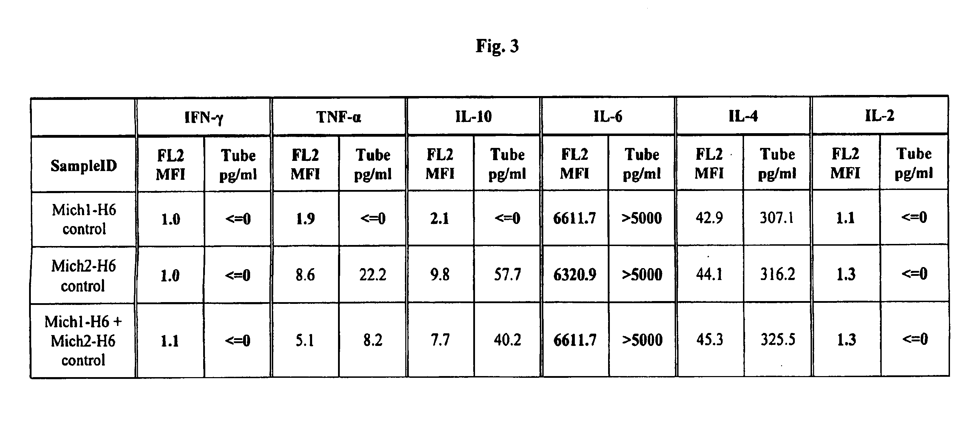

As shown in Fig. 3, Mich1-H6 cells did not produce IFN-γ, TNF-α, IL-10 and IL-2. Mich2-H6 cells did not produce IFN-γ and IL-2. As expected, these cytokines were also not seen in culture media of mixtures of both cell lines. However, low quantities of TNF-α and IL-10 were secreted by Mich2-H6 cells. Both cell lines produced IL-6 and IL-4 in very high and moderate quantities, respectively. These two cytokines were produced at comparable levels by each cell line.

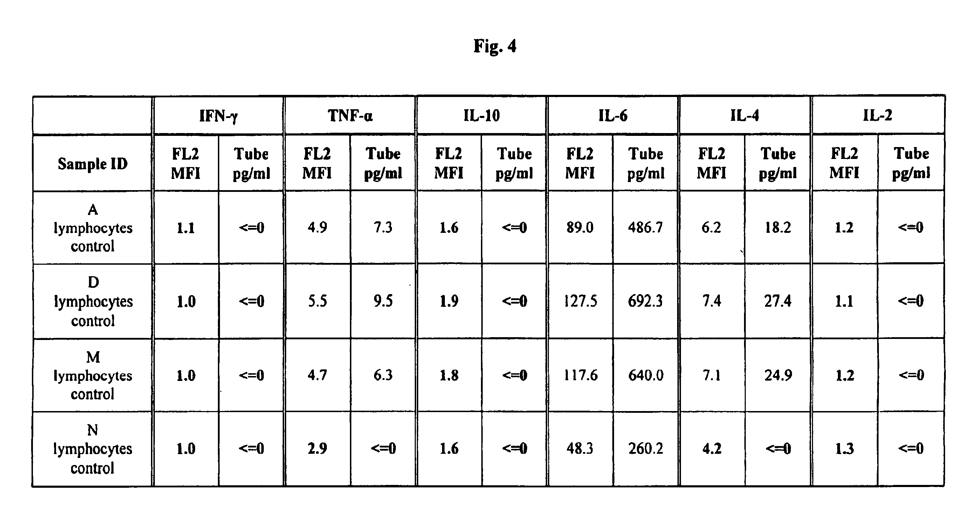

In Fig. 4 the results of cytokine production by PBLCs are shown. The PBLC did not produce IFN-γ, IL-10 or IL-2. The remaining cytokines examined, i.e. TNF-α, IL-6 and IL-4, were produced by PBLC at comparable levels.

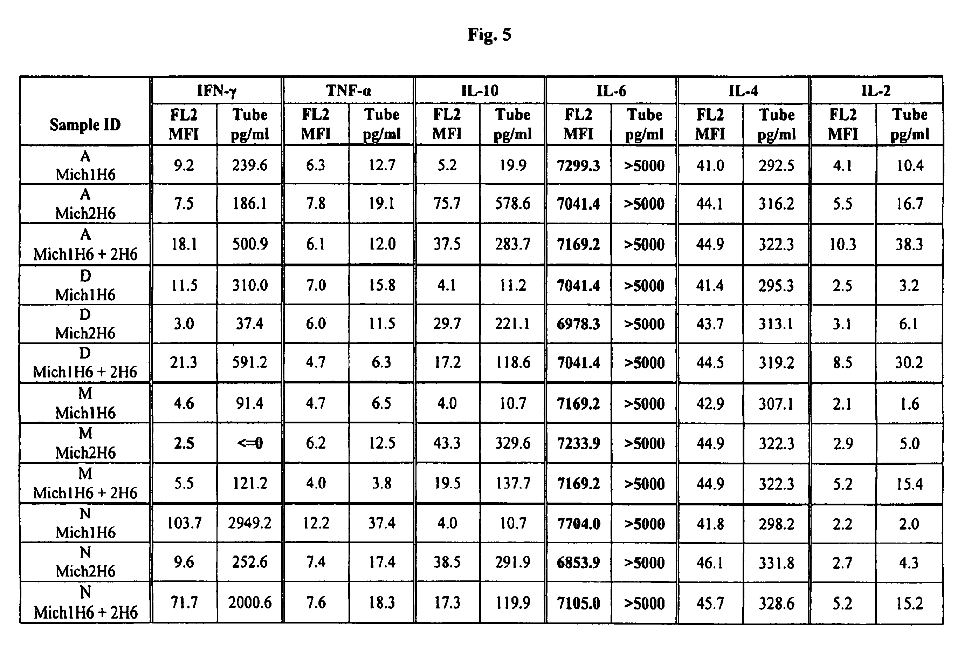

Fig. 5 shows results of the effect of Mich1-H6 and Mich2-H6 cells alone and in combination on PBLC cytokine secretion. Incubation of PBLC with melanoma cell lines led to the modulation of cytokine production by PBLC for most of the cytokines studied. Only IL-4 and likely IL-6 secretion were not affected. In PBLCs from all four donors, a synergistic effect of a mixture of both cell lines as compared to each cell line used separately was observed with respect to IL-2 production. A similar synergistic effect was seen in 3 out of 4 donors on IFN-γ production. An inhibitory effect of a mixture of cell lines over cells used separately was observed with regard to TNF-α production in PBLCs from 3 out of 4 donors. The production of IL-10 caused by the mixture of Mich1-H6 and Mich2-H6 was in between the values observed when PBLCs were stimulated by Mich1-H6 or Mich2-H6 separately.

### Summary of results from Example 5

The results obtained demonstrate that the mixture of two allogenic melanoma cell lines displays different biological effects on cytokine secretion by PBLC isolated from healthy donors as compared to each cell line used separately. These effects proved to be synergistic on the increased production of IL-2 and INF-γ and inhibitory on TNF-α secretion. Increased IL-2 and INF-γ production indicate the beneficial shift towards a Th1 immune response. Decreased TNF-α secretion requires further studies since the cytokine quantities detected were near to the lower detection limit of the assay.

In conclusion, the combination of Mich1-H6 and Mich2-H6 cells increases *in vitro* immunogeneicity of the vaccine as compared to each cell line used alone by shifting the immune response towards a Th1 type as demonstrated by the synergistic effect on IL-2 and INF-γ production by PBLC. Thus, the genetic modification of tumour cells with a designer cytokine, e.g. a hypercytokine such as H6, and the combination of multiple tumour cell lines increases the therapeutic potential of an allogenic vaccine.

### REFERENCES

Bazan JF. Structural design and molecular evolution of a cytokine receptor superfamily. Proc Natl Acad Sci U S A. 1990 Se; 87(18):6934-8.
Bravo J, Heath JK. Receptor recognition by gp130 cytokines. EMBO J. 2000 Jun 1;19(11):2399-411.
Brudno M., Bioinformatics 2003, 19 Suppl 1:I54-I62
Carlezon WA Jr, Nestler EJ, Neve RL. Herpes simplex virus-mediated gene transfer as a tool for neuropsychiatric research. Crit Rev Neurobiol. 2000; 14(1):47-67.
Carter PJ, Samulski RJ. Adeno-associated viral vectors as gene delivery vehicles. Int J Mol Med. 2000 Jul;6(1): 17-27.
Chebath J. et al. Interleukin-6 receptor-interleukin-6 fusion proteins with enhanced interleukin-6 type pleiotropic activities. European Cytokine Network, Dec 1997, vol. 8, no. 4, pp. 359-365
Chang LJ, Gay EE. The molecular genetics of lentiviral vectors--current and future perspectives. Curr Gene Ther. 2001 Sep;1(3):237-51.
Davis S, Aldrich TH, Valenzuela DM, Wong VV, Furth ME, Squinto SP, Yancopoulos GD. The receptor for ciliary neurotrophic factor. Science. 1991 Jul 5;253(5015):59-63.
Deller MC, Hudson KR, Ikemizu S, Bravo J, Jones EY, Heath JK. Crystal structure and functional dissection of the cytostatic cytokine oncostatin M. Structure Fold Des. 2000 Aug 15;8(8):863-74.
Fischer M, Goldschmitt J, Peschel C, Brakenhoff JP, Kallen KJ, Wollmer A, Grotzinger J, Rose-John S. I. A bioactive designer cytokine for human hematopoietic progenitor cell expansion. Nat Biotechnol. 1997 Feb; 15(2): 142-5.
Heinrich PC, Behrmann I, Haan S, Hermanns HM, Muller-Newen G, Schaper F. Principles of interleukin (IL)-6-type cytokine signalling and its regulation. Biochem J. 2003 Aug 15;374(Pt 1):1-20.
Heller M.J. DNA microarray technology: devices, systems, and applications. Annu. Rev. Biomed. Eng. 2002, 4:129-53
Kallen K.J. et al. The therapeutic potential of interleukin-6 hyperagonists and antagonists. Expert Opinion on Investigational Drugs (1997 Mar), vol. 6, No. 3, pp. 237-266
Kubler H, Vieweg J. Vaccines in renal cell carcinoma. Semin. Oncol. (2006) 33(5):614-624.
Lieschke GJ, Rao PK, Gately MK, Mulligan RG. Bioactive murine and human interleukin-12 fusion proteins which retain antitumour activity in vivo. Nat Biotechnol. 1997 Jan; 15(1): 35-40.
Lindemann D, Patriquin E, Feng S, Mulligan RC. Versatile retrovirus vector systems for regulated gene expression in vitro and in vivo. Mol Med. 1997 Jul;3(7):466-76.
Marz P, Otten U, Rose-John S. Neural activities of IL-6-type cytokines often depend on soluble cytokine receptors. Eur J Neurosci. 1999 Sep;11 (9):2995-3004.
McDonald NQ, Panayotatos N, Hendrickson WA. Crystal structure of dimeric human ciliary neurotrophic factor determined by MAD phasing. EMBO J. 1995 Jun 15;14(12):2689-99.
Pflanz S, Tacken I, Grotzinger J, Jacques Y, Minvielle S, Dahmen H, Heinrich PC, Muller-Newen G. A fusion protein of interleukin-11 and soluble interleukin-11 receptor acts as a superagonist on cells expressing gp130. FEBS Lett. 1999 Apr 30;450(1-2): 117-22.
Robinson RC, Grey LM, Staunton D, Vankelecom H, Vennallis AB, Moreau JF, Stuart DI, Heath JK, Jones EY. The crystal structure and biological function of leukemia inhibitory factor: implications for receptor binding. Cell. 1994 Jul 1;77(7): 1101-16.
Rose-John S, Schooltink H, Lenz D, Hipp E, Dufhues G, Schmitz H, Schiel X, Hirano T, Kishimoto T, Heinrich PC. Studies on the structure and regulation of the human hepatic interleukin-6 receptor. Eur J Biochem. 1990 May 31;190(1):79-83.
Russo G., Zegar C. and Giordano A. Advantages and limitations of microarray technology in human cancer. Oncogene. 2003, 22:6497-507
Somers W, Stahl M, Seehra JS. 1.9 A crystal structure of interleukin 6: implications for a novel mode of receptor dimerization and signaling. EMBO J. 1997 Mar 3;16(5):989-97.
Springer ML, Chen AS, Kraft PE, Bednarski M, Blau HM. VEGF gene delivery to muscle: potential role for vasculogenesis in adults. Mol Cell 1998 Nov;2(5):549-58.
Thompson, J. D., Higgins, D. G., Gibson, T. J. (1994) Nucleic Acids Res. 22, 4673-80
Türeci O., Sahin U. and Pfreundschuh M. Serological analysis of human tumor antigens: molecular definition and implications. Mol Med Today. 1997, 3:342-349
Wells JA. Binding in the growth hormone receptor complex. Proc Natl Acad Sci U S A. 1996 Jan 9;93(1):1-6.
Wiznerowicz M, Fong AZ, Mackiewicz A, Hawley RG. Double-copy bicistronic retroviral vector platform for gene therapy and tissue engineering: application to melanoma vaccine development. GeneTher. 1997 Oct;4(10):1061-8.

## Claims

1. A composition comprising:
1) one or more first tumour cells modified to express a first hyper-cytokine, and
2) one or more second tumour cells modified to express a second hyper-cytokine, wherein said second cells are different from said first cells, wherein the first and second hyper-cytokine is a fusion protein comprising a cytokine receptor and a cytokine and wherein the second cells have a different HLA type than the first cells.

2. The composition of claim 1,
wherein the cytokine receptor is independently selected from
(a) the group consisting of sIL-6R, sIL-11R, OSM-R, CNTF-R, and CT-I-R; or
(b) a polypeptide exhibiting at least 90% sequence identity to a polypeptide according to (a); and
wherein the cytokine is independently selected from
(c) the group consisting of IL-6, IL-11, OSM, CNTF, and CT-I; or
(d) a polypeptide exhibiting at least 90% sequence identity to a polypeptide according to (c),
wherein the hyper-cytokine has hyper-cytokine activity.

3. The composition of claim 1 or 2, wherein the cytokine receptor and the cytokine are directly linked or linked by a peptide linker.

4. The composition of any one of claims 1 to 3, wherein the hyper-cytokine is a fusion protein comprising
(a) an IL-6R part exhibiting at least 90% sequence identity to human soluble IL-6 receptor (sIL-6R), wherein said sequence identity is calculated over the entire length of the polypeptide sequence from P113 to A323 of SEQ ID NO: 1,
(b) an IL-6 part exhibiting at least 90% sequence identity to human interleukin-6 (IL-6), wherein said sequence identity is calculated over the entire length of the polypeptide sequence from P29 to M212 of SEQ ID NO: 2, and
(c) optionally a peptide linker;
having hyper-cytokine activity
or
(a) an IL-11R part exhibiting at least 90% sequence identity to human soluble IL-11 receptor (sIL-11R), wherein said sequence identity is calculated over the entire length of the polypeptide sequence from M 1 to Q365 of SEQ ID NO: 3,
(b) an IL-11 part exhibiting at least 90% sequence identity to human interleukin-11 (IL-11), wherein said sequence identity is calculated over the entire length of the polypeptide sequence from A19 to L 199 of SEQ ID NO: 4, and
(c) optionally a peptide linker
having hyper-cytokine activity.

5. The composition of any one of claims 1 to 3, wherein the hyper-cytokine is
(a) a polypeptide having the amino acid sequence according to SEQ ID NO: 5, SEQ ID NO: 6, SEQ ID NO: 7, SEQ ID NO: 8 or SEQ ID NO: 9; or
(b) a polypeptide exhibiting at least 90% sequence identity to a polypeptide according to (a) and having hyper-cytokine activity.

6. The composition of any of claims 1 to 5, wherein the first cell and/or the second cells are allogenic cells.

7. The composition of any of claims 1 to 6, wherein the tumour cells are independently selected for the first and second cells from the group consisting of melanoma cells, renal carcinoma cells, prostate cancer cells, colon cancer cells, lung cancer cells, pancreas cancer cells, liver cancer cells, brain cancer cells, head and neck cancer cells, and sarcoma cells.

8. The composition of any of claims 1 to 7, wherein the first tumour cells are Mich1, deposited under accession number DSM ACC2837 with the "Deutsche Sammlung von Mikroorganismen und Zellkulturen" (DSMZ) and the second tumour cells are Mich2, deposited under accession number DSM ACC2838 with DSMZ.

9. The composition of any one of claims 1 to 7, wherein the first cells are Mich 1-H6, deposited under accession number DSM ACC2839 with DSMZ.

10. The composition of any one of claims 1 to 7 or 9, wherein the second cells are Mich2-H6, deposited under accession number DSM ACC 2840 with DSMZ.

11. The composition of any one of claims 1 to 10, wherein the proliferation of the first and/or the second cells has been inhibited.

12. A composition according to any one of claims 1 to 11 for use in medicine.

13. A pharmaceutical composition comprising a composition according to any one of claims 1 to 12 additionally comprising pharmaceutically acceptable diluents, carriers, excipients, fillers, binders, lubricants, glidants, disintegrants, adsorbents, and/or preservatives.

14. The composition according to any one of claims 1 to 12 or the pharmaceutical composition according to claim 13 for use in the treatment or prevention of cancer.

15. The composition or pharmaceutical composition of claim 14, wherein the cancer is selected from the group consisting of gastrointestinal or colorectal tract, liver, pancreas, kidney, bladder, prostate, endometrium, head and neck cancer, ovary, testes, prostate, skin, eye, melanoma, dysplastic oral mucosa, invasive oral cancer, small cell and non-small cell lung cancer, hormone-dependent breast cancer, hormone independent breast cancer, transitional and squamous cell cancer, neurological malignancy, including neuroblastoma, endocrinological tumour, hematologic neoplasia, carcinoma in situ, hyperplastic lesion, adenoma, and fibroma.

16. The composition or pharmaceutical composition of claim 14 or 15, wherein the patient to be treated has a partially or completely different HLA type than the first cells and/or the second cells.

## Patentansprüche

1. Zusammensetzung umfassend:
1) eine oder mehrere erste Tumorzellen, die modifiziert ist/sind, um ein erstes Hypercytokin zu exprimieren, und
2) eine oder mehrere zweite Tumorzellen, die modifiziert ist/sind, um ein zweites Hypercytokin zu exprimieren, wobei die zweiten Zellen sich von den ersten Zellen unterscheiden, wobei das erste und das zweite Hypercytokin ein Fusionsprotein ist, das einen Cytokinrezeptor und ein Cytokin umfasst, und wobei die zweiten Zellen einen anderen HLA-Typ aufweisen als die ersten Zellen.

2. Zusammensetzung nach Anspruch 1,
wobei der Cytokinrezeptor unabhängig ausgewählt ist aus
(a) der Gruppe bestehend aus sIL-6R, sIL-11R, OSM-R, CNTF-R und CT-I-R; oder
(b) einem Polypeptid, das wenigstens 90% Sequenzidentität zu einem Polypeptid gemäß (a) aufweist; und
wobei das Cytokin unabhängig ausgewählt ist aus
(c) der Gruppe bestehend aus IL-6, IL-11, OSM, CNTF und CT-I; oder
(d) einem Polypeptid, das wenigstens 90% Sequenzidentität zu einem Polypeptid gemäß (c) aufweist,
wobei das Hypercytokin Hypercytokin-Aktivität aufweist.

3. Zusammensetzung nach Anspruch 1 oder 2, wobei der Cytokinrezeptor und das Cytokin direkt oder über einen Peptidlinker verknüpft sind.

4. Zusammensetzung nach einem der Ansprüche 1 bis 3, wobei das Hypercytokin ein Fusionsprotein ist, umfassend
(a) einen IL-6R-Teil, der wenigstens 90% Sequenzidentität zum humanen, löslichen IL-6-Rezeptor (sIL-6R) aufweist, wobei die Sequenzidentität über die gesamte Länge der Polypeptidsequenz von P113 bis A323 von SEQ ID NO: 1 berechnet wird,
(b) einen IL-6-Teil, der wenigstens 90% Sequenzidentität zu humanem Interleukin-6 (IL-6) aufweist, wobei die Sequenzidentität über die gesamte Länge der Polypeptidsequenz von P29 bis M212 von SEQ ID NO: 2 berechnet wird, und
(c) optional einen Peptidlinker;
welches Hypercytokin-Aktivität aufweist,
oder
(a) einen IL-11R-Teil, der wenigstens 90% Sequenzidentität zum humanen, löslichen IL-11-Rezeptor (sIL-11R) aufweist, wobei die Sequenzidentität über die gesamte Länge der Polypeptidsequenz von M1 bis Q365 von SEQ ID NO: 3 berechnet wird,
(b) einen IL-11-Teil, der wenigstens 90% Sequenzidentität zu humanem Interleukin-11 (IL-11) aufweist, wobei die Sequenzidentität über die gesamte Länge der Polypeptidsequenz von A 19 bis L 199 von SEQ ID NO: 4 berechnet wird, und
(c) optional einen Peptidlinker,
welches Hypercytokin-Aktivität aufweist.

5. Zusammensetzung nach einem der Ansprüche 1 bis 3, wobei das Hypercytokin
(a) ein Polypeptid mit der Aminosäuresequenz gemäß SEQ ID NO: 5, SEQ ID NO: 6, SEQ ID NO: 7, SEQ ID NO: 8 oder SEQ ID NO: 9;
oder
(b) ein Polypeptid, das wenigstens 90% Sequenzidentität zu einem Polypeptid gemäß (a) aufweist und das Hypercytokin-Aktivität hat,
ist.

6. Zusammensetzung nach einem der Ansprüche 1 bis 5, wobei die erste Zelle und/oder die zweiten Zellen allogene Zellen sind.

7. Zusammensetzung nach einem der Ansprüche 1 bis 6, wobei die Tumorzellen für die ersten und zweiten Zellen unabhängig ausgewählt werden aus der Gruppe bestehend aus Melanomzellen, Nierenkarzinomzellen, Prostatakrebszellen, Dickdarmkrebszellen, Lungenkrebszellen, Bauchspeicheldrüsenkrebszellen, Leberkrebszellen, Gehirnkrebszellen, Kopf-Hals-Karzinomzellen und Sarkomzellen.

8. Zusammensetzung nach einem der Ansprüche 1 bis 7, wobei die ersten Tumorzellen Mich 1 sind, die unter der Zugangsnummer DSM ACC2837 bei der Deutschen Sammlung von Mikroorganismen und Zellkulturen (DSMZ) hinterlegt worden sind, und die zweiten Tumorzellen Mich2 sind, die unter der Zugangsnummer DSM ACC2838 bei der DSMZ hinterlegt worden sind.

9. Zusammensetzung nach einem der Ansprüche 1 bis 7, wobei die ersten Zellen Michl-H6 sind, die unter der Zugangsnummer DSM ACC2839 bei der DSMZ hinterlegt worden sind.

10. Zusammensetzung nach einem der Ansprüche 1 bis 7 oder 9, wobei die zweiten Zellen Mich2-H6 sind, die unter der Zugangsnummer DSM ACC2840 bei der DSMZ hinterlegt worden sind.

11. Zusammensetzung nach einem der Ansprüche 1 bis 10, wobei die Proliferation der ersten und/oder der zweiten Zellen inhibiert worden ist.

12. Zusammensetzung gemäß einem der Ansprüche 1 bis 11 zur Verwendung in der Medizin.

13. Pharmazeutische Zusammensetzung, die eine Zusammensetzung gemäß einem der Ansprüche 1 bis 12 umfasst, und die zusätzlich pharmazeutisch verträgliche Verdünnungsmittel, Trägersubstanzen, Hilfsstoffe, Füllmittel, Bindemittel, Schmiermittel, Fließregulierungsmittel, Sprengmittel, Adsorptionsmittel und/oder Konservierungsstoffe umfasst.

14. Zusammensetzung gemäß einem der Ansprüche 1 bis 12 oder die pharmazeutische Zusammensetzung gemäß Anspruch 13 zur Verwendung in der Behandlung von oder der Vorbeugung gegen Krebs.

15. Zusammensetzung oder pharmazeutische Zusammensetzung nach Anspruch 14, wobei der Krebs ausgewählt ist aus der Gruppe bestehend aus Krebs des Gastrointestinal- oder Colorektaltrakts, Leberkrebs, Bauchspeicheldrüsenkrebs, Nierenkrebs, Blasenkrebs, Prostatakrebs, Krebs des Endometriums, Kopf-Hals-Karzinom, Eierstockkrebs, Hodenkrebs, Prostatakrebs, Hautkrebs, Augenkrebs, Melanom, Mundschleimhautdysplasie, invasivem Mundhöhlenkarzinom, kleinzelligem und nicht-kleinzelligem Lungenkarzinom, hormonabhängigem Brustkrebs, hormonunabhängigem Brustkrebs, Übergangszellkarzinom und Plattenepithelkarzinom, neurologischem Malignom einschließlich Neuroblastom, endokrinologischem Tumor, hämatologischer Neoplasie, Karzinom in situ, hyperplastischer Läsion, Adenom und Fibrom.

16. Zusammensetzung oder pharmazeutische Zusammensetzung nach Anspruch 14 oder 15, wobei der zu behandelnde Patient einen teilweise oder vollständig anderen HLA-Typ aufweist als die ersten Zellen und/oder die zweiten Zellen.

## Revendications

1. Composition comprenant:
1) une ou plusieurs premières cellules tumorales modifiées pour exprimer une première hyper-cytokine, et
2) une ou plusieurs deuxièmes cellules tumorales modifiées pour exprimer une deuxième hyper-cytokine, tandis que lesdites deuxièmes cellules sont différentes desdites premières cellules, tandis que la première et la deuxième hyper-cytokine est une protéine de fusion comprenant un récepteur de cytokine et une cytokine, et tandis que les deuxièmes cellules ont un type de HLA différent de celui des premières cellules.

2. Composition selon la revendication 1,
dans laquelle le récepteur de cytokine est indépendamment choisi parmi
(a) le groupe consistant en sIL-6R, sIL-11R, OSM-R, CNTF-R, et CT-I-R; ou
(b) un polypeptide présentant au moins 90% d'identité de séquence vis-à-vis d'un polypeptide selon (a); et
tandis que la cytokine est indépendamment choisie parmi
(c) le groupe consistant en IL-6, IL-11, OSM, CNTF, et CT-I; ou
(d) un polypeptide présentant au moins 90% d'identité de séquence vis-à-vis d'un polypeptide selon (c),
tandis que l'hyper-cytokine a une activité d'hyper-cytokine.

3. Composition selon la revendication 1 ou 2, dans laquelle le récepteur de cytokine et la cytokine sont liés directement ou liés par un lieur peptidique.

4. Composition selon l'une quelconque des revendications 1 à 3, dans laquelle l'hyper-cytokine est une protéine de fusion comprenant
(a) une partie IL-6R présentant au moins 90% d'identité de séquence vis-à-vis du récepteur d'IL-6 soluble humain (sIL-6R), tandis que ladite identité de séquence est calculée sur la longueur totale de la séquence polypeptidique allant de P113 à A323 de SEQ ID NO: 1,
(b) une partie IL-6 présentant au moins 90% d'identité de séquence vis-à-vis de l'interleukine-6 humaine (IL-6), tandis que ladite identité de séquence est calculée sur la longueur totale de la séquence polypeptidique allant de P29 à M212 de SEQ ID NO: 2, et
(c) en option, un lieur peptidique;
ayant une activité d'hyper-cytokine
ou
(a) une partie IL-11R présentant au moins 90% d'identité de séquence vis-à-vis du récepteur d'IL-11 soluble humain (sIL-11R), tandis que ladite identité de séquence est calculée sur la longueur totale de la séquence polypeptidique allant de M1 à Q365 de SEQ ID NO: 3,
(b) une partie IL-11 présentant au moins 90% d'identité de séquence vis-à-vis de l'interleukine-11 humaine (IL-11), tandis que ladite identité de séquence est calculée sur la longueur totale de la séquence polypeptidique allant de A19 à L199 de SEQ ID NO: 4, et
(c) en option, un lieur peptidique
ayant une activité d'hyper-cytokine.

5. Composition selon l'une quelconque des revendications 1 à 3, dans laquelle l'hyper-cytokine est
(a) un polypeptide ayant la séquence d'acides aminés selon SEQ ID NO: 5, SEQ ID NO: 6, SEQ ID NO: 7, SEQ ID NO: 8 ou SEQ ID NO: 9; ou
(b) un polypeptide présentant au moins 90% d'identité de séquence vis-à-vis d'un polypeptide selon (a) et ayant une activité d'hyper-cytokine.

6. Composition selon l'une quelconque des revendications 1 à 5, dans laquelle la première cellule et/ ou les deuxièmes cellules sont des cellules allogéniques.

7. Composition selon l'une quelconque des revendications 1 à 6, dans laquelle les cellules tumorales sont indépendamment choisies pour les premières et deuxièmes cellules dans le groupe consistant en cellules de mélanome, cellules de carcinome rénal, cellules de cancer de la prostate, cellules de cancer du côlon, cellules de cancer du poumon, cellules de cancer du pancréas, cellules de cancer du foie, cellules de cancer du cerveau, cellules de cancer de la tête et du cou, et cellules de sarcome.

8. Composition selon l'une quelconque des revendications 1 à 7, dans laquelle les premières cellules tumorales sont Mich1, déposée sous le numéro d'accès DSM ACC2837 auprès la "Deutsche Sammlung von Mikroorganismen und Zellkulturen" (DSMZ) et les deuxièmes cellules tumorales sont Mich2, déposée sous le numéro d'accès DSM ACC2838 auprès de la DSMZ.

9. Composition selon l'une quelconque des revendications 1 à 7, dans laquelle les premières cellules sont Mich1-H6, déposée sous le numéro d'accès DSM ACC2839 auprès de la DSMZ.

10. Composition selon l'une quelconque des revendications 1 à 7 ou 9, dans laquelle les deuxièmes cellules sont Mich2-H6, déposée sous le numéro d'accès DSM ACC2840 auprès de la DSMZ.

11. Composition selon l'une quelconque des revendications 1 à 10, dans laquelle la prolifération des premières et/ ou des deuxièmes cellules a été inhibée.

12. Composition selon l'une quelconque des revendications 1 à 11 pour utilisation en médecine.

13. Composition pharmaceutique comprenant une composition selon l'une quelconque des revendications 1 à 12, comprenant en outre des diluants, supports, excipients, charges, liants, lubrifiants, agents de glissement, agents de désintégration, adsorbants et/ou conservateurs, pharmaceutiquement acceptables.

14. Composition selon l'une quelconque des revendications 1 à 12 ou composition pharmaceutique selon la revendication 13, pour utilisation dans le traitement ou la prévention du cancer.

15. Composition ou composition pharmaceutique selon la revendication 14, dans laquelle le cancer est choisi dans le groupe consistant en cancer gastro-intestinal ou des voies colo-rectales, du foie, du pancréas, du rein, de la vessie, de la prostate, de l'endomètre, de la tête et du cou, mélanome des ovaires, des testicules, de la prostate, de la peau, des yeux, muqueuse orale dysplasique, cancer buccal invasif, cancer du poumon à petites cellules et à cellules non-petites, cancer du sein dépendant des hormones, cancer du sein indépendant des hormones, cancer des cellules transitoires et squameuses, caractère malin neurologique, notamment neuroblastome, tumeur endocrinologique, néoplasie hématologique, carcinome *in situ,* lésion hyperplasique, adénome, et fibrome.

16. Composition ou composition pharmaceutiquement selon la revendication 14 ou 15, dans laquelle le patient à traiter présente un type de HLA partiellement ou totalement différent de celui des premières cellules et/ou des deuxièmes cellules.


### SEQUENCE LISTING

<110> AGIRX Ltd.
<120> vaccine Compositions
<130> 494-17 PCT2
<140> 2009-01-29
   <141>
<150> PCT/EP2008/000780
   <151> 2008-01-31
<160> 15
<170> Patent in version 3.3
<210> 1
   <211> 468
   <212> PRT
   <213> Homo sapiens
<400> 1
   Met Leu Ala Val Gly Cys Ala Leu Leu Ala Ala Leu Leu Ala Ala Pro
   1               5                   10                  15
   Gly Ala Ala Leu Ala Pro Arg Arg Cys Pro Ala Gln Glu Val Ala Arg
               20                  25                  30
   Gly Val Leu Thr Ser Leu Pro Gly Asp Ser Val Thr Leu Thr Cys Pro
           35                  40                  45
   Gly Val Glu Pro Glu Asp Asn Ala Thr Val His Trp Val Leu Arg Lys
       50                  55                  60
   Pro Ala Ala Gly Ser His Pro Ser Arg Trp Ala Gly Met Gly Arg Arg
   65                  70                  75                  80
   Leu Leu Leu Arg Ser Val Gln Leu His Asp Ser Gly Asn Tyr Ser Cys
                   85                  90                  95
   Tyr Arg Ala Gly Arg Pro Ala Gly Thr Val His Leu Leu Val Asp Val
               100                 105                 110
   Pro Pro Glu Glu Pro Gln Leu Ser Cys Phe Arg Lys Ser Pro Leu Ser
           115                 120                 125
   Asn Val Val Cys Glu Trp Gly Pro Arg Ser Thr Pro Ser Leu Thr Thr
       130                 135                 140
   Lys Ala Val Leu Leu Val Arg Lys Phe Gln Asn Ser Pro Ala Glu Asp
   145                 150                 155                 160
   Phe Gln Glu Pro Cys Gln Tyr Ser Gln Glu Ser Gln Lys Phe Ser Cys
                   165                 170                 175
   Gln Leu Ala Val Pro Glu Gly Asp Ser Ser Phe Tyr Ile Val Ser Met
   180 185 190
   Cys Val Ala Ser Ser Val Gly Ser Lys Phe Ser Lys Thr Gln Thr Phe
   195 200 205
   Gln Gly Cys Gly Ile Leu Gln Pro Asp Pro Pro Ala Asn Ile Thr Val
   210 215 220
   Thr Ala Val Ala Arg Asn Pro Arg Trp Leu Ser Val Thr Trp Gln Asp
   225 230 235 240
   Pro His Ser Trp Asn Ser Ser Phe Tyr Arg Leu Arg Phe Glu Leu Arg
   245 250 255
   Tyr Arg Ala Glu Arg Ser Lys Thr Phe Thr Thr Trp Met Val Lys Asp
   260 265 270
   Leu Gln His His Cys Val Ile His Asp Ala Trp Ser Gly Leu Arg His
   275 280 285
   Val Val Gln Leu Arg Ala Gln Glu Glu Phe Gly Gln Gly Glu Trp Ser
   290 295 300
   Glu Trp Ser Pro Glu Ala Met Gly Thr Pro Trp Thr Glu Ser Arg Ser
   305 310 315 320
   Pro Pro Ala Glu Asn Glu Val Ser Thr Pro Met Gln Ala Leu Thr Thr
   325 330 335
   Asn Lys Asp Asp Asp Asn Ile Leu Phe Arg Asp Ser Ala Asn Ala Thr
   340 345 350
   Ser Leu Pro Val Gln Asp Ser Ser Ser Val Pro Leu Pro Thr Phe Leu
   355 360 365
   Val Ala Gly Gly Ser Leu Ala Phe Gly Thr Leu Leu Cys Ile Ala Ile
   370 375 380
   Val Leu Arg Phe Lys Lys Thr Trp Lys Leu Arg Ala Leu Lys Glu Gly
   385 390 395 400
   Lys Thr Ser Met His Pro Pro Tyr Ser Leu Gly Gln Leu Val Pro Glu
   405 410 415
   Arg Pro Arg Pro Thr Pro Val Leu Val Pro Leu Ile Ser Pro Pro Val
   420 425 430
   Ser Pro Ser Ser Leu Gly Ser Asp Asn Thr Ser Ser His Asn Arg Pro
   435 440 445
   Asp Ala Arg Asp Pro Arg Ser Pro Tyr Asp Ile Ser Asn Thr Asp Tyr
   450 455 460
   Phe Phe Pro Arg
   465
<210> 2
   <211> 212
   <212> PRT
   <213> Homo sapiens
<400> 2
   Met Asn Ser Phe Ser Thr Ser Ala Phe Gly Pro Val Ala Phe Ser Leu
   1 5 10 15
   Gly Leu Leu Leu Val Leu Pro Ala Ala Phe Pro Ala Pro Val Pro Pro
   20 25 30
   Gly Glu Asp Ser Lys Asp Val Ala Ala Pro His Arg Gln Pro Leu Thr
   35 40 45
   Ser Ser Glu Arg Ile Asp Lys Gln Ile Arg Tyr Ile Leu Asp Gly Ile
   50 55 60
   Ser Ala Leu Arg Lys Glu Thr Cys Asn Lys Ser Asn Met Cys Glu Ser
   65 70 75 80
   Ser Lys Glu Ala Leu Ala Glu Asn Asn Leu Asn Leu Pro Lys Met Ala
   85 90 95
   Glu Lys Asp Gly Cys Phe Gln Ser Gly Phe Asn Glu Glu Thr Cys Leu
   100 105 110
   Val Lys Ile Ile Thr Gly Leu Leu Glu Phe Glu Val Tyr Leu Glu Tyr
   115 120 125
   Leu Gln Asn Arg Phe Glu Ser Ser Glu Glu Gln Ala Arg Ala Val Gln
   130 135 140
   Met Ser Thr Lys Val Leu Ile Gln Phe Leu Gln Lys Lys Ala Lys Asn
   145 150 155 160
   Leu Asp Ala Ile Thr Thr Pro Asp Pro Thr Thr Asn Ala Ser Leu Leu
   165 170 175
   Thr Lys Leu Gln Ala Gln Asn Gln Trp Leu Gln Asp Met Thr Thr His
   180 185 190
   Leu Ile Leu Arg Ser Phe Lys Glu Phe Leu Gln Ser Ser Leu Arg Ala
   195 200 205
   Leu Arg Gln Met
   210
<210> 3
   <211> 422
   <212> PRT
   <213> Homo sapiens
<400> 3
   Met Gly Ser Ser Cys Ser Gly Leu Ser Arg Val Leu Val Ala Val Ala
   1               5                   10                  15
   Thr Ala Leu Val Ser Ala Ser Ser Pro Cys Pro Gln Ala Trp Gly Pro
               20                  25                  30
   Pro Gly Val Gln Tyr Gly Gln Pro Gly Arg Ser Val Lys Leu Cys Cys
           35                  40                  45
   Pro Gly Val Thr Ala Gly Asp Pro Val Ser Trp Phe Arg Asp Gly Glu
       50                  55                  60
   Pro Lys Leu Leu Gln Gly Pro Asp Ser Gly Leu Gly His Glu Leu Val
   65                  70                  75                  80
   Leu Ala Gln Ala Asp Ser Thr Asp Glu Gly Thr Tyr Ile Cys Gln Thr
                   85                  90                  95
   Leu Asp Gly Ala Leu Gly Gly Thr Val Thr Leu Gln Leu Gly Tyr Pro
               100                 105                 110
   Pro Ala Arg Pro Val Val Ser Cys Gln Ala Ala Asp Tyr Glu Asn Phe
           115                 120                 125
   Ser Cys Thr Trp Ser Pro Ser Gln Ile Ser Gly Leu Pro Thr Arg Tyr
       130                 135                 140
   Leu Thr Ser Tyr Arg Lys Lys Thr Val Leu Gly Ala Asp Ser Gln Arg
   145                 150                 155                 160
   Arg Ser Pro Ser Thr Gly Pro Trp Pro Cys Pro Gln Asp Pro Leu Gly
                   165                 170                 175
   Ala Ala Arg Cys Val Val His Gly Ala Glu Phe Trp Ser Gln Tyr Arg
               180                 185                 190
   Ile Asn Val Thr Glu Val Asn Pro Leu Gly Ala Ser Thr Arg Leu Leu
           195                 200                 205
   Asp Val Ser Leu Gln Ser Ile Leu Arg Pro Asp Pro Pro Gln Gly Leu
       210                 215                 220
   Arg Val Glu Ser Val Pro Gly Tyr Pro Arg Arg Leu Arg Ala Ser Trp
   225                 230                 235                 240
   Thr Tyr Pro Ala Ser Trp Pro Cys Gln Pro His Phe Leu Leu Lys Phe
                   245                 250                 255
   Arg Leu Gln Tyr Arg Pro Ala Gln His Pro Ala Trp Ser Thr Val Glu
               260                 265                 270
   Pro Ala Gly Leu Glu Glu Val Ile Thr Asp Ala Val Ala Gly Leu Pro
           275                 280                 285
   His Ala Val Arg Val Ser Ala Arg Asp Phe Leu Asp Ala Gly Thr Trp
       290                 295                 300
   Ser Thr Trp Ser Pro Glu Ala Trp Gly Thr Pro Ser Thr Gly Thr Ile
   305                 310                 315                 320
   Pro Lys Glu Ile Pro Ala Trp Gly Gln Leu His Thr Gln Pro Glu Val
                   325                 330                 335
   Glu Pro Gln Val Asp Ser Pro Ala Pro Pro Arg Pro Ser Leu Gln Pro
               340                 345                 350
   His Pro Arg Leu Leu Asp His Arg Asp Ser Val Glu Gln Val Ala Val
           355                 360                 365
   Leu Ala Ser Leu Gly Ile Leu Ser Phe Leu Gly Leu Val Ala Gly Ala
       370                 375                 380
   Leu Ala Leu Gly Leu Trp Leu Arg Leu Arg Arg Gly Gly Lys Asp Gly
   385                 390                 395                 400
   Ser Pro Lys Pro Gly Phe Leu Ala Ser Val Ile Pro Val Asp Arg Arg
                   405                 410                 415
   Pro Gly Ala Pro Asn Leu
               420
<210> 4
   <211> 199
   <212> PRT
   <213> Homo sapiens
<400> 4
   Met Asn Cys Val Cys Arg Leu Val Leu Val Val Leu Ser Leu Trp Pro
   1               5                   10                  15
   Asp Thr Ala Val Ala Pro Gly Pro Pro Pro Gly Pro Pro Arg Val Ser
               20                  25                  30
   Pro Asp Pro Arg Ala Glu Leu Asp Ser Thr Val Leu Leu Thr Arg Ser
           35                  40                  45
   Leu Leu Ala Asp Thr Arg Gln Leu Ala Ala Gln Leu Arg Asp Lys Phe
   50 55 60
   Pro Ala Asp Gly Asp His Asn Leu Asp Ser Leu Pro Thr Leu Ala Met
   65 70 75 80
   Ser Ala Gly Ala Leu Gly Ala Leu Gln Leu Pro Gly Val Leu Thr Arg
   85 90 95
   Leu Arg Ala Asp Leu Leu Ser Tyr Leu Arg His Val Gln Trp Leu Arg
   100 105 110
   Arg Ala Gly Gly Ser Ser Leu Lys Thr Leu Glu Pro Glu Leu Gly Thr
   115 120 125
   Leu Gln Ala Arg Leu Asp Arg Leu Leu Arg Arg Leu Gln Leu Leu Met
   130 135 140
   Ser Arg Leu Ala Leu Pro Gln Pro Pro Pro Asp Pro Pro Ala Pro Pro
   145 150 155 160
   Leu Ala Pro Pro Ser Ser Ala Trp Gly Gly Ile Arg Ala Ala His Ala
   165 170 175
   Ile Leu Gly Gly Leu His Leu Thr Leu Asp Trp Ala Val Arg Gly Leu
   180 185 190
   Leu Leu Leu Lys Thr Arg Leu
   195
<210> 5
   <211> 408
   <212> PRT
   <213> Artificial
<220>
   <223> Hypercytokine H-IL-6, Fischer et al. 1997
<400>
   Pro Pro Glu Glu Pro Gln Leu Ser Cys Phe Arg Lys Ser Pro Leu Ser
   1 5 10 15
   Asn Val Val Cys Glu Trp Gly Pro Arg Ser Thr Pro Ser Leu Thr Thr
   20 25 30
   Lys Ala Val Leu Leu Val Arg Lys Phe Gln Asn Ser Pro Ala Glu Asp
   35 40 45
   Phe Gln Glu Pro Cys Gln Tyr Ser Gln Glu Ser Gln Lys Phe Ser Cys
   50 55 60
   Gln Leu Ala Val Pro Glu Gly Asp Ser Ser Phe Tyr Ile Val Ser Met
   65                  70                  75                  80
   Cys Val Ala Ser Ser Val Gly Ser Lys Phe Ser Lys Thr Gln Thr Phe
                   85                  90                  95
   Gln Gly Cys Gly Ile Leu Gln Pro Asp Pro Pro Ala Asn Ile Thr Val
               100                 105                 110
   Thr Ala Val Ala Arg Asn Pro Arg Trp Leu Ser Val Thr Trp Gln Asp
           115                 120                 125
   Pro His Ser Trp Asn Ser Ser Phe Tyr Arg Leu Arg Phe Glu Leu Arg
       130                 135                 140
   Tyr Arg Ala Glu Arg Ser Lys Thr Phe Thr Thr Trp Met Val Lys Asp
   145                 150                 155                 160
   Leu Gln His His Cys Val Ile His Asp Ala Trp Ser Gly Leu Arg His
                   165                 170                 175
   Val Val Gln Leu Arg Ala Gln Glu Glu Phe Gly Gln Gly Glu Trp Ser
               180                 185                 190
   Glu Trp Ser Pro Glu Ala Met Gly Thr Pro Trp Thr Glu Ser Arg Ser
           195                 200                 205
   Pro Pro Ala Arg Gly Gly Gly Gly Ser Gly Gly Gly Gly Ser Val Glu
       210                 215                 220
   Pro Val Pro Pro Gly Glu Asp Ser Lys Asp Val Ala Ala Pro His Arg
   225                 230                 235                 240
   Gln Pro Leu Thr Ser Ser Glu Arg Ile Asp Lys Gln Ile Arg Tyr Ile
                   245                 250                 255
   Leu Asp Gly Ile Ser Ala Leu Arg Lys Glu Thr Cys Asn Lys Ser Asn
               260                 265                 270
   Met Cys Glu Ser Ser Lys Glu Ala Leu Ala Glu Asn Asn Leu Asn Leu
           275                 280                 285
   Pro Lys Met Ala Glu Lys Asp Gly Cys Phe Gln Ser Gly Phe Asn Glu
       290                 295                 300
   Glu Thr Cys Leu Val Lys Ile Ile Thr Gly Leu Leu Glu Phe Glu Val
   305                 310                 315                 320
   Tyr Leu Glu Tyr Leu Gln Asn Arg Phe Glu Ser Ser Glu Glu Gln Ala
                   325                 330                 335
   Arg Ala Val Gln Met Ser Thr Lys Val Leu Ile Gln Phe Leu Gln Lys
   340 345 350
   Lys Ala Lys Asn Leu Asp Ala Ile Thr Thr Pro Asp Pro Thr Thr Asn
   355 360 365
   Ala Ser Leu Leu Thr Lys Leu Gln Ala Gln Asn Gln Trp Leu Gln Asp
   370 375 380
   Met Thr Thr His Leu Ile Leu Arg Ser Phe Lys Glu Phe Leu Gln Ser
   385 390 395 400
   Ser Leu Arg Ala Leu Arg Gln Met
   405
<210> 6
   <211> 543
   <212> PRT
   <213> Artificial
<220>
   <223> sIL-6R/IL-6 with linker EFM, Chebath et al. 1997
<400> 6
   Met Leu Ala Val Gly Cys Ala Leu Leu Ala Ala Leu Leu Ala Ala Pro
   1 5 10 15
   Gly Ala Ala Leu Ala Pro Arg Arg Cys Pro Ala Gln Glu Val Ala Arg
   20 25 30
   Gly Val Leu Thr Ser Leu Pro Gly Asp Ser Val Thr Leu Thr Cys Pro
   35 40 45
   Gly Val Glu Pro Glu Asp Asn Ala Thr Val His Trp Val Leu Arg Lys
   50 55 60
   Pro Ala Ala Gly Ser His Pro Ser Arg Trp Ala Gly Met Gly Arg Arg
   65 70 75 80
   Leu Leu Leu Arg Ser Val Gln Leu His Asp Ser Gly Asn Tyr Ser Cys
   85 90 95
   Tyr Arg Ala Gly Arg Pro Ala Gly Thr Val His Leu Leu Val Asp Val
   100 105 110
   Pro Pro Glu Glu Pro Gln Leu Ser Cys Phe Arg Lys Ser Pro Leu Ser
   115 120 125
   Asn Val Val Cys Glu Trp Gly Pro Arg Ser Thr Pro Ser Leu Thr Thr
   130 135 140
   Lys Ala Val Leu Leu Val Arg Lys Phe Gln Asn Ser Pro Ala Glu Asp
   145 150 155 160
   Phe Gln Glu Pro Cys Gln Tyr Ser Gln Glu Ser Gln Lys Phe Ser Cys
                   165                 170                 175
   Gln Leu Ala Val Pro Glu Gly Asp Ser Ser Phe Tyr Ile Val Ser Met
               180                 185                 190
   Cys Val Ala Ser Ser Val Gly Ser Lys Phe Ser Lys Thr Gln Thr Phe
           195                 200                 205
   Gln Gly Cys Gly Ile Leu Gln Pro Asp Pro Pro Ala Asn Ile Thr Val
       210                 215                 220
   Thr Ala Val Ala Arg Asn Pro Arg Trp Leu Ser Val Thr Trp Gln Asp
   225                 230                 235                 240
   Pro His Ser Trp Asn Ser Ser Phe Tyr Arg Leu Arg Phe Glu Leu Arg
                   245                 250                 255
   Tyr Arg Ala Glu Arg Ser Lys Thr Phe Thr Thr Trp Met Val Lys Asp
               260                 265                 270
   Leu Gln His His Cys Val Ile His Asp Ala Trp Ser Gly Leu Arg His
           275                 280                 285
   Val Val Gln Leu Arg Ala Gln Glu Glu Phe Gly Gln Gly Glu Trp Ser
       290                 295                 300
   Glu Trp Ser Pro Glu Ala Met Gly Thr Pro Trp Thr Glu Ser Arg Ser
   305                 310                 315                 320
   Pro Pro Ala Glu Asn Glu Val Ser Thr Pro Met Gln Ala Leu Thr Thr
                   325                 330                 335
   Asn Lys Asp Asp Asp Asn Ile Leu Phe Arg Asp Ser Ala Asn Ala Thr
               340                 345                 350
   Ser Leu Pro Val Glu Phe Met Pro Val Pro Pro Gly Glu Asp Ser Lys
           355                 360                 365
   Asp Val Ala Ala Pro His Arg Gln Pro Leu Thr Ser Ser Glu Arg Ile
       370                 375                 380
   Asp Lys Gln Ile Arg Tyr Ile Leu Asp Gly Ile Ser Ala Leu Arg Lys
   385                 390                 395                 400
   Glu Thr Cys Asn Lys Ser Asn Met Cys Glu Ser Ser Lys Glu Ala Leu
                   405                 410                 415
   Ala Glu Asn Asn Leu Asn Leu Pro Lys Met Ala Glu Lys Asp Gly Cys
               420                 425                 430
   Phe Gln Ser Gly Phe Asn Glu Glu Thr Cys Leu Val Lys Ile Ile Thr
           435                 440                 445
   Gly Leu Leu Glu Phe Glu Val Tyr Leu Glu Tyr Leu Gln Asn Arg Phe
       450                 455                 460
   Glu Ser Ser Glu Glu Gln Ala Arg Ala Val Gln Met Ser Thr Lys Val
   465                 470                 475                 480
   Leu Ile Gln Phe Leu Gln Lys Lys Ala Lys Asn Leu Asp Ala Ile Thr
                   485                 490                 495
   Thr Pro Asp Pro Thr Thr Asn Ala Ser Leu Leu Thr Lys Leu Gln Ala
               500                 505                 510
   Gln Asn Gln Trp Leu Gln Asp Met Thr Thr His Leu Ile Leu Arg Ser
           515                 520                 525
   Phe Lys Glu Phe Leu Gln Ser Ser Leu Arg Ala Leu Arg Gln Met
       530                 535                 540
<210> 7
   <211> 553
   <212> PRT
   <213> Artificial
<220>
   <223> sIL-6R/IL-6 with linker EFGAGLVLGGQFM, Chebath et al. 1997
<400> 7
   Met Leu Ala Val Gly Cys Ala Leu Leu Ala Ala Leu Leu Ala Ala Pro
   1               5                   10                  15
   Gly Ala Ala Leu Ala Pro Arg Arg Cys Pro Ala Gln Glu Val Ala Arg
               20                  25                  30
   Gly Val Leu Thr Ser Leu Pro Gly Asp Ser Val Thr Leu Thr Cys Pro
           35                  40                  45
   Gly Val Glu Pro Glu Asp Asn Ala Thr Val His Trp Val Leu Arg Lys
       50                  55                  60
   Pro Ala Ala Gly Ser His Pro Ser Arg Trp Ala Gly Met Gly Arg Arg
   65                  70                  75                  80
   Leu Leu Leu Arg Ser Val Gln Leu His Asp Ser Gly Asn Tyr Ser Cys
                   85                  90                  95
   Tyr Arg Ala Gly Arg Pro Ala Gly Thr Val His Leu Leu Val Asp Val
               100                 105                 110
   Pro Pro Glu Glu Pro Gln Leu Ser Cys Phe Arg Lys Ser Pro Leu Ser
   115 120 125
   Asn Val Val Cys Glu Trp Gly Pro Arg Ser Thr Pro Ser Leu Thr Thr
   130 135 140
   Lys Ala Val Leu Leu Val Arg Lys Phe Gln Asn Ser Pro Ala Glu Asp
   145 150 155 160
   Phe Gln Glu Pro Cys Gln Tyr Ser Gln Glu Ser Gln Lys Phe Ser Cys
   165 170 175
   Gln Leu Ala Val Pro Glu Gly Asp Ser Ser Phe Tyr Ile Val Ser Met
   180 185 190
   Cys Val Ala Ser Ser Val Gly Ser Lys Phe Ser Lys Thr Gln Thr Phe
   195 200 205
   Gln Gly Cys Gly Ile Leu Gln Pro Asp Pro Pro Ala Asn Ile Thr Val
   210 215 220
   Thr Ala Val Ala Arg Asn Pro Arg Trp Leu Ser Val Thr Trp Gln Asp
   225 230 235 240
   Pro His Ser Trp Asn Ser Ser Phe Tyr Arg Leu Arg Phe Glu Leu Arg
   245 250 255
   Tyr Arg Ala Glu Arg Ser Lys Thr Phe Thr Thr Trp Met Val Lys Asp
   260 265 270
   Leu Gln His His Cys Val Ile His Asp Ala Trp Ser Gly Leu Arg His
   275 280 285
   Val Val Gln Leu Arg Ala Gln Glu Glu Phe Gly Gln Gly Glu Trp Ser
   290 295 300
   Glu Trp Ser Pro Glu Ala Met Gly Thr Pro Trp Thr Glu Ser Arg Ser
   305 310 315 320
   Pro Pro Ala Glu Asn Glu Val Ser Thr Pro Met Gln Ala Leu Thr Thr
   325 330 335
   Asn Lys Asp Asp Asp Asn Ile Leu Phe Arg Asp Ser Ala Asn Ala Thr
   340 345 350
   Ser Leu Pro Val Glu Phe Gly Ala Gly Leu Val Leu Gly Gly Gln Phe
   355 360 365
   Met Pro Val Pro Pro Gly Glu Asp Ser Lys Asp Val Ala Ala Pro His
   370 375 380
   Arg Gln Pro Leu Thr Ser Ser Glu Arg Ile Asp Lys Gln Ile Arg Tyr
   385 390 395 400
   Ile Leu Asp Gly Ile Ser Ala Leu Arg Lys Glu Thr Cys Asn Lys Ser
   405 410 415
   Asn Met Cys Glu Ser Ser Lys Glu Ala Leu Ala Glu Asn Asn Leu Asn
   420 425 430
   Leu Pro Lys Met Ala Glu Lys Asp Gly Cys Phe Gln Ser Gly Phe Asn
   435 440 445
   Glu Glu Thr Cys Leu Val Lys Ile Ile Thr Gly Leu Leu Glu Phe Glu
   450 455 460
   Val Tyr Leu Glu Tyr Leu Gln Asn Arg Phe Glu Ser Ser Glu Glu Gln
   465 470 475 480
   Ala Arg Ala Val Gln Met Ser Thr Lys Val Leu Ile Gln Phe Leu Gln
   485 490 495
   Lys Lys Ala Lys Asn Leu Asp Ala Ile Thr Thr Pro Asp Pro Thr Thr
   500 505 510
   Asn Ala Ser Leu Leu Thr Lys Leu Gln Ala Gln Asn Gln Trp Leu Gln
   515 520 525
   Asp Met Thr Thr His Leu Ile Leu Arg Ser Phe Lys Glu Phe Leu Gln
   530 535 540
   Ser Ser Leu Arg Ala Leu Arg Gln Met
   545 550
<210> 8
   <211> 540
   <212> PRT
   <213**>** Artificial
<220>
   <223> sIL-6R/IL-6 without linker, Chebath et al., 1997
<400> 8
   Met Leu Ala Val Gly Cys Ala Leu Leu Ala Ala Leu Leu Ala Ala Pro
   1 5 10 15
   Gly Ala Ala Leu Ala Pro Arg Arg Cys Pro Ala Gln Glu Val Ala Arg
   20 25 30
   Gly Val Leu Thr Ser Leu Pro Gly Asp Ser Val Thr Leu Thr Cys Pro
   35 40 45
   Gly Val Glu Pro Glu Asp Asn Ala Thr Val His Trp Val Leu Arg Lys
   50 55 60
   Pro Ala Ala Gly Ser His Pro Ser Arg Trp Ala Gly Met Gly Arg Arg
   65                  70                  75                  80
   Leu Leu Leu Arg Ser Val Gln Leu His Asp Ser Gly Asn Tyr Ser Cys
                   85                  90                  95
   Tyr Arg Ala Gly Arg Pro Ala Gly Thr Val His Leu Leu Val Asp Val
               100                 105                 110
   Pro Pro Glu Glu Pro Gln Leu Ser Cys Phe Arg Lys Ser Pro Leu Ser
           115                 120                 125
   Asn Val Val Cys Glu Trp Gly Pro Arg Ser Thr Pro Ser Leu Thr Thr
       130                 135                 140
   Lys Ala Val Leu Leu Val Arg Lys Phe Gln Asn Ser Pro Ala Glu Asp
   145                 150                 155                 160
   Phe Gln Glu Pro Cys Gln Tyr Ser Gln Glu Ser Gln Lys Phe Ser Cys
                   165                 170                 175
   Gln Leu Ala Val Pro Glu Gly Asp Ser Ser Phe Tyr Ile Val Ser Met
               180                 185                 190
   Cys Val Ala Ser Ser Val Gly Ser Lys Phe Ser Lys Thr Gln Thr Phe
           195                 200                 205
   Gln Gly Cys Gly Ile Leu Gln Pro Asp Pro Pro Ala Asn Ile Thr Val
       210                 215                 220
   Thr Ala Val Ala Arg Asn Pro Arg Trp Leu Ser Val Thr Trp Gln Asp
   225                 230                 235                 240
   Pro His Ser Trp Asn Ser Ser Phe Tyr Arg Leu Arg Phe Glu Leu Arg
                   245                 250                 255
   Tyr Arg Ala Glu Arg Ser Lys Thr Phe Thr Thr Trp Met Val Lys Asp
               260                 265                 270
   Leu Gln His His Cys Val Ile His Asp Ala Trp Ser Gly Leu Arg His
           275                 280                 285
   Val Val Gln Leu Arg Ala Gln Glu Glu Phe Gly Gln Gly Glu Trp Ser
       290                 295                 300
   Glu Trp Ser Pro Glu Ala Met Gly Thr Pro Trp Thr Glu Ser Arg Ser
   305                 310                 315                 320
   Pro Pro Ala Glu Asn Glu Val Ser Thr Pro Met Gln Ala Leu Thr Thr
                   325                 330                 335
   Asn Lys Asp Asp Asp Asn Ile Leu Phe Arg Asp Ser Ala Asn Ala Thr
   340 345 350
   Ser Leu Pro Val Pro Val Pro Pro Gly Glu Asp Ser Lys Asp Val Ala
   355 360 365
   Ala Pro His Arg Gln Pro Leu Thr Ser Ser Glu Arg Ile Asp Lys Gln
   370 375 380
   Ile Arg Tyr Ile Leu Asp Gly Ile Ser Ala Leu Arg Lys Glu Thr Cys
   385 390 395 400
   Asn Lys Ser Asn Met Cys Glu Ser Ser Lys Glu Ala Leu Ala Glu Asn
   405 410 415
   Asn Leu Asn Leu Pro Lys Met Ala Glu Lys Asp Gly Cys Phe Gln Ser
   420 425 430
   Gly Phe Asn Glu Glu Thr Cys Leu Val Lys Ile Ile Thr Gly Leu Leu
   435 440 445
   Glu Phe Glu Val Tyr Leu Glu Tyr Leu Gln Asn Arg Phe Glu Ser Ser
   450 455 460
   Glu Glu Gln Ala Arg Ala Val Gln Met Ser Thr Lys Val Leu Ile Gln
   465 470 475 480
   Phe Leu Gln Lys Lys Ala Lys Asn Leu Asp Ala Ile Thr Thr Pro Asp
   485 490 495
   Pro Thr Thr Asn Ala Ser Leu Leu Thr Lys Leu Gln Ala Gln Asn Gln
   500 505 510
   Trp Leu Gln Asp Met Thr Thr His Leu Ile Leu Arg Ser Phe Lys Glu
   515 520 525
   Phe Leu Gln Ser Ser Leu Arg Ala Leu Arg Gln Met
   530 535 540
<210> 9
   <211> 520
   <212> PRT
   <213> Artificial
<220>
   <223> Fusion of human sIL-6R/IL-6 with 13 aa linker
<400> 9
   Met Leu Ala Val Gly Cys Ala Leu Leu Ala Ala Leu Leu Ala Ala Pro
   1 5 10 15
   Gly Ala Ala Leu Ala Pro Arg Arg Cys Pro Ala Gln Glu Val Ala Arg
   20 25 30
   Gly Val Leu Thr Ser Leu Pro Gly Asp Ser Val Thr Leu Thr Cys Pro
   35 40 45
   Gly Val Glu Pro Glu Asp Asn Ala Thr Val His Trp Val Leu Arg Lys
   50 55 60
   Pro Ala Ala Gly Ser His Pro Ser Arg Trp Ala Gly Met Gly Arg Arg
   65 70 75 80
   Leu Leu Leu Arg Ser Val Gln Leu His Asp Ser Gly Asn Tyr Ser Cys
   85 90 95
   Tyr Arg Ala Gly Arg Pro Ala Gly Thr Val His Leu Leu Val Asp Val
   100 105 110
   Pro Pro Glu Glu Pro Gln Leu Ser Cys Phe Arg Lys Ser Pro Leu Ser
   115 120 125
   Asn Val Val Cys Glu Trp Gly Pro Arg Ser Thr Pro Ser Leu Thr Thr
   130 135 140
   Lys Ala Val Leu Leu Val Arg Lys Phe Gln Asn Ser Pro Ala Glu Asp
   145 150 155 160
   Phe Gln Glu Pro Cys Gln Tyr Ser Gln Glu Ser Gln Lys Phe Ser Cys
   165 170 175
   Gln Leu Ala Val Pro Glu Gly Asp Ser Ser Phe Tyr Ile Val Ser Met
   180 185 190
   Cys Val Ala Ser Ser Val Gly Ser Lys Phe Ser Lys Thr Gln Thr Phe
   195 200 205
   Gln Gly Cys Gly Ile Leu Gln Pro Asp Pro Pro Ala Asn Ile Thr Val
   210 215 220
   Thr Ala Val Ala Arg Asn Pro Arg Trp Leu Ser Val Thr Trp Gln Asp
   225 230 235 240
   Pro His Ser Trp Asn Ser Ser Phe Tyr Arg Leu Arg Phe Glu Leu Arg
   245 250 255
   Tyr Arg Ala Glu Arg Ser Lys Thr Phe Thr Thr Trp Met Val Lys Asp
   260 265 270
   Leu Gln His His Cys Val Ile His Asp Ala Trp Ser Gly Leu Arg His
   275 280 285
   Val Val Gln Leu Arg Ala Gln Glu Glu Phe Gly Gln Gly Glu Trp Ser
       290                 295                 300
   Glu Trp Ser Pro Glu Ala Met Gly Thr Pro Trp Thr Glu Ser Arg Ser
   305                 310                 315                 320
   Pro Pro Ala Arg Gly Gly Gly Gly Ser Gly Gly Gly Gly Ser Val Glu
                   325                 330                 335
   Pro Val Pro Pro Gly Glu Asp Ser Lys Asp Val Ala Ala Pro His Arg
               340                 345                 350
   Gln Pro Leu Thr Ser Ser Glu Arg Ile Asp Lys Gln Ile Arg Tyr Ile
           355                 360                 365
   Leu Asp Gly Ile Ser Ala Leu Arg Lys Glu Thr Cys Asn Lys Ser Asn
       370                 375                 380
   Met Cys Glu Ser Ser Lys Glu Ala Leu Ala Glu Asn Asn Leu Asn Leu
   385                 390                 395                 400
   Pro Lys Met Ala Glu Lys Asp Gly Cys Phe Gln Ser Gly Phe Asn Glu
                   405                 410                 415
   Glu Thr Cys Leu Val Lys Ile Ile Thr Gly Leu Leu Glu Phe Glu Val
               420                 425                 430
   Tyr Leu Glu Tyr Leu Gln Asn Arg Phe Glu Ser Ser Glu Glu Gln Ala
           435                 440                 445
   Arg Ala Val Gln Met Ser Thr Lys Val Leu Ile Gln Phe Leu Gln Lys
       450                 455                 460
   Lys Ala Lys Asn Leu Asp Ala Ile Thr Thr Pro Asp Pro Thr Thr Asn
   465                 470                 475                 480
   Ala Ser Leu Leu Thr Lys Leu Gln Ala Gln Asn Gln Trp Leu Gln Asp
                   485                 490                 495
   Met Thr Thr His Leu Ile Leu Arg Ser Phe Lys Glu Phe Leu Gln Ser
               500                 505                 510
   Ser Leu Arg Ala Leu Arg Gln Met
           515                 520
<210> 10
   <211> 525
   <212> PRT
   <213> Artificial
<220>
   <223> Fusion of human sIL-6R/IL-6 with 18 aa linker
<400> 10
   Met Leu Ala Val Gly Cys Ala Leu Leu Ala Ala Leu Leu Ala Ala Pro
   1 5 10 15
   Gly Ala Ala Leu Ala Pro Arg Arg Cys Pro Ala Gln Glu Val Ala Arg
   20 25 30
   Gly Val Leu Thr Ser Leu Pro Gly Asp Ser Val Thr Leu Thr Cys Pro
   35 40 45
   Gly Val Glu Pro Glu Asp Asn Ala Thr Val His Trp Val Leu Arg Lys
   50 55 60
   Pro Ala Ala Gly Ser His Pro Ser Arg Trp Ala Gly Met Gly Arg Arg
   65 70 75 80
   Leu Leu Leu Arg Ser Val Gln Leu His Asp Ser Gly Asn Tyr Ser Cys
   85 90 95
   Tyr Arg Ala Gly Arg Pro Ala Gly Thr Val His Leu Leu Val Asp Val
   100 105 110
   Pro Pro Glu Glu Pro Gln Leu Ser Cys Phe Arg Lys Ser Pro Leu Ser
   115 120 125
   Asn Val Val Cys Glu Trp Gly Pro Arg Ser Thr Pro Ser Leu Thr Thr
   130 135 140
   Lys Ala Val Leu Leu Val Arg Lys Phe Gln Asn Ser Pro Ala Glu Asp
   145 150 155 160
   Phe Gln Glu Pro Cys Gln Tyr Ser Gln Glu Ser Gln Lys Phe Ser Cys
   165 170 175
   Gln Leu Ala Val Pro Glu Gly Asp Ser Ser Phe Tyr Ile Val Ser Met
   180 185 190
   Cys Val Ala Ser Ser Val Gly Ser Lys Phe Ser Lys Thr Gln Thr Phe
   195 200 205
   Gln Gly Cys Gly Ile Leu Gln Pro Asp Pro Pro Ala Asn Ile Thr Val
   210 215 220
   Thr Ala Val Ala Arg Asn Pro Arg Trp Leu Ser Val Thr Trp Gln Asp
   225 230 235 240
   Pro His Ser Trp Asn Ser Ser Phe Tyr Arg Leu Arg Phe Glu Leu Arg
   245 250 255
   Tyr Arg Ala Glu Arg Ser Lys Thr Phe Thr Thr Trp Met Val Lys Asp
   260 265 270
   Leu Gln His His Cys Val Ile His Asp Ala Trp Ser Gly Leu Arg His
           275                 280                 285
   Val Val Gln Leu Arg Ala Gln Glu Glu Phe Gly Gln Gly Glu Trp Ser
       290                 295                 300
   Glu Trp Ser Pro Glu Ala Met Gly Thr Pro Trp Thr Glu Ser Arg Ser
   305                 310                 315                 320
   Pro Pro Ala Arg Gly Gly Gly Gly Ser Gly Gly Gly Gly Ser Gly Gly
                   325                 330                 335
   Gly Gly Ser Val Glu Pro Val Pro Pro Gly Glu Asp Ser Lys Asp Val
               340                 345                 350
   Ala Ala Pro His Arg Gln Pro Leu Thr Ser Ser Glu Arg Ile Asp Lys
           355                 360                 365
   Gln Ile Arg Tyr Ile Leu Asp Gly Ile Ser Ala Leu Arg Lys Glu Thr
       370                 375                 380
   Cys Asn Lys Ser Asn Met Cys Glu Ser Ser Lys Glu Ala Leu Ala Glu
   385                 390                 395                 400
   Asn Asn Leu Asn Leu Pro Lys Met Ala Glu Lys Asp Gly Cys Phe Gln
                   405                 410                 415
   Ser Gly Phe Asn Glu Glu Thr Cys Leu Val Lys Ile Ile Thr Gly Leu
               420                 425                 430
   Leu Glu Phe Glu Val Tyr Leu Glu Tyr Leu Gln Asn Arg Phe Glu Ser
           435                 440                 445
   Ser Glu Glu Gln Ala Arg Ala Val Gln Met Ser Thr Lys Val Leu Ile
       450                 455                 460
   Gln Phe Leu Gln Lys Lys Ala Lys Asn Leu Asp Ala Ile Thr Thr Pro
   465                 470                 475                 480
   Asp Pro Thr Thr Asn Ala Ser Leu Leu Thr Lys Leu Gln Ala Gln Asn
                   485                 490                 495
   Gln Trp Leu Gln Asp Met Thr Thr His Leu Ile Leu Arg Ser Phe Lys
               500                 505                 510
   Glu Phe Leu Gln Ser Ser Leu Arg Ala Leu Arg Gln Met
           515                 520                 525
<210> 11
   <211> 546
   <212> PRT
   <213> Artificial
<220>
   <223> Hypercytokine H11, WO2005/113591
<400> 11
<210> 12
   <211> 13
   <212> PRT
   <213> Artificial
<220>
   <223> Peptide linker
<400> 12
   Glu Phe Gly Ala Gly Leu Val Leu Gly Gly Gln Phe Met
   1 5 10
<210> 13
   <211> 13
   <212> PRT
   <213> Artificial
<220>
   <223> Peptide linker
<400> 13
   Arg Gly Gly Gly Gly Ser Gly Gly Gly Gly Ser Val Glu
   1 5 10
<210> 14
   <211> 18
   <212> PRT
   <213> Artificial
<220>
   <223> Peptide linker
<400> 14
   Arg Gly Gly Gly Gly Ser Gly Gly Gly Gly Ser Gly Gly Gly Gly Ser
   1 5 10 15
   Val Glu
<210> 15
   <211> 3792
   <212> DNA
   <213> Artificial
<220>
   <223> Expression cassette comprising the CMV immediate early promoter directing expression of Hyper-IL-6 and Neomycin separated by an IRES
<400> 15
   gactagttat taatagtaat caattacggg gtcattagtt catagcccat atatggagtt 60
   ccgcgttaca taacttacgg taaatggccc gcctggctga ccgcccaacg acccccgccc 120
   attgacgtca ataatgacgt atgttcccat agtaacgcca atagggactt tccattgacg 180
   tcaatgggtg gagtatttac ggtaaactgc ccacttggca gtacatcaag tgtatcatat 240
   gccaagtacg ccccctattg acgtcaatga cggtaaatgg cccgcctggc attatgccca 300
   gtacatgacc ttatgggact ttcctacttg gcagtacatc tacgtattag tcatcgctat 360
   taccatggtg atgcggtttt ggcagtacat caatgggcgt ggatagcggt ttgactcacg 420
   gggatttcca agtctccacc ccattgacgt caatgggagt ttgttttggc accaaaatca 480
   acgggacttt ccaaaatgtc gtaacaactc cgccccattg acgcaaatgg gcggtaggcg 540
   tgtacggtgg gaggtctata taagcagagc tcgtttagtg aaccgtcaga tcgcctggag 600
   acgccatcca cgctgttttg acctccatag aagacaccgg gactggacaa ttcgagctcg 660
   gtacccaatt cgagctcgcc cggggatgcg gccgctcgac gcatggagtg gtagccgagg 720
   aggaagcatg ctggccgtcg gctgcgcgct gctggctgcc ctgctggccg cgccgggagc 780
   ggcgctggcc ccaaggcgct gccctgcgca ggaggtggcg agaggcgtgc tgaccagtct 840
   gccaggagac agcgtgactc tgacctgccc gggggtagag ccggaagaca atgccactgt 900
   tcactgggtg ctcaggaagc cggctgcagg ctcccacccc agcagatggg ctggcatggg 960
   aaggaggctg ctgctgaggt cggtgcagct ccacgactct ggaaactatt catgctaccg 1020
   ggccggccgc ccagctggga ctgtgcactt gctggtggat gttccccccg aggagcccca 1080
   gctctcctgc ttccggaaga gccccctcag caatgttgtt tgtgagtggg gtcctcggag 1140
   caccccatcc ctgacgacaa aggctgtgct cttggtgagg aagtttcaga acagtccggc 1200
   cgaagacttc caggagccgt gccagtattc ccaggagtcc cagaagttct cctgccagtt 1260
   agcagtcccg gagggagaca gctctttcta catagtgtcc atgtgcgtcg ccagtagtgt 1320
   cgggagcaag ttcagcaaaa ctcaaacctt tcagggttgt ggaatcttgc agcctgatcc 1380
   gcctgccaac atcacagtca ctgccgtggc cagaaacccc cgctggctca gtgtcacctg 1440
   gcaagacccc cactcctgga actcatcttt ctacagacta cggtttgagc tcagatatcg 1500
   ggctgaacgg tcaaagacat tcacaacatg gatggtcaag gacctccagc atcactgtgt 1560
   catccacgac gcctggagcg gcctgaggca cgtggtgcag cttcgtgccc aggaggagtt 1620
   cgggcaaggc gagtggagcg agtggagccc ggaggccatg ggcacgcctt ggacagaatc 1680
   caggagtcct ccagctcgag gaggtggagg ttctggaggt ggaggttctg tcgagccagt 1740
   acccccagga gaagattcca aagatgtagc cgccccacac agacagccac tcacctcttc 1800
   agaacgaatt gacaaacaaa ttcggtacat cctcgacggc atctcagccc tgagaaagga 1860
   gacatgtaac aagagtaaca tgtgtgaaag cagcaaagag gcactggcag aaaacaacct 1920
   gaaccttcca aagatggctg aaaaagatgg atgcttccaa tctggattca atgaggagac 1980
   ttgcctggtg aaaatcatca ctggtctttt ggagtttgag gtatacctag agtacctcca 2040
   gaacagattt gagagtagtg aggaacaagc cagagctgtg cagatgagta caaaagtcct   2100
   gatccagttc ctgcagaaaa aggcaaagaa tctagatgca ataaccaccc ctgacccaac   2160
   cacaaatgcc agcctgctga cgaagctgca ggcacagaac cagtggctgc aggacatgac   2220
   aactcatctc attctgcgca gctttaagga gttcctgcag tccagcctga gggctcttcg   2280
   gcaaatgtag catgggcacg tcgagggatc ttccatacct accagttctg cgcctgcagg   2340
   tcgcggccgc atcctctaga gtcgacccga ccgcggacgc tcgactgcag gaattaattc   2400
   cgcccccccc cccccccccc cctaacgtta ctggccgaag ccgcttggaa taaggccggt   2460
   gtgcgtttgt ctatatgtta ttttccacca tattgccgtc ttttggcaat gtgagggccc   2520
   ggaaacctgg ccctgtcttc ttgacgagca ttcctagggg tctttcccct ctcgccaaag   2580
   gaatgcaagg tctgttgaat gtcgtgaagg aagcagttcc tctggaagct tcttgaagac   2640
   aaacaacgtc tgtagcgacc ctttgcaggc agcggaaccc cccacctggc gacaggtgcc   2700
   tctgcggcca aaagccacgt gtataagata cacctgcaaa ggcggcacaa ccccagtgcc   2760
   acgttgtgag ttggatagtt gtggaaagag tcaaatggct ctcctcaagc gtattcaaca   2820
   aggggctgaa ggatgcccag aaggtacccc attgtatggg atctgatctg gggcctcggt   2880
   gcacatgctt tacatgtgtt tagtcgaggt taaaaaaacg tctaggcccc ccgaaccacg   2940
   gggacgtggt tttcctttga aaaacacgat gataagcttg ccacaaccat gggatcggcc   3000
   attgaacaag atggattgca cgcaggttct ccggccgctt gggtggagag gctattcggc   3060
   tatgactggg cacaacagac aatcggctgc tctgatgccg ccgtgttccg gctgtcagcg   3120
   caggggcgcc cggttctttt tgtcaagacc gacctgtccg gtgccctgaa tgaactgcag   3180
   gacgaggcag cgcggctatc gtggctggcc acgacgggcg ttccttgcgc agctgtgctc   3240
   gacgttgtca ctgaagcggg aagggactgg ctgctattgg gcgaagtgcc ggggcaggat   3300
   ctcctgtcat ctcaccttgc tcctgccgag aaagtatcca tcatggctga tgcaatgcgg   3360
   cggctgcata cgcttgatcc ggctacctgc ccattcgacc accaagcgaa acatcgcatc   3420
   gagcgagcac gtactcggat ggaagccggt cttgtcgatc aggatgatct ggacgaagag   3480
   catcaggggc tcgcgccagc cgaactgttc gccaggctca aggcgcgcat gcccgacggc   3540
   gaggatctcg tcgtgaccca tggcgatgcc tgcttgccga atatcatggt ggaaaatggc   3600
   cgcttttctg gattcatcga ctgtggccgg ctgggtgtgg cggaccgcta tcaggacata   3660
   gcgttggcta cccgtgatat tgctgaagag cttggcggcg aatgggctga ccgcttcctc   3720
   gtgctttacg gtatcgccgc tcccgattcg cagcgcatcg ccttctatcg ccttcttgac   3780
   gagttcttct ga                                                       3792